# EUROPEAN PATENT APPLICATION

(11) **EP 4 209 591 A1**
(43) Date of publication of application: **12.07.2023**
(21) Application number: 21863668.6
(22) Date of filing: 02.09.2021
(51) Int. Cl.: C12N 15/113, C12N 15/85, C12N 5/10, C07K 16/00, A01K 67/027

(54) **METHOD FOR PREPARING NON-HUMAN MAMMAL OR PROGENY THEREOF, AND USE THEREOF**

(30) Priority: 04.09.2020 CN 202010924095
(71) Applicant: Rengene Biotechnology Co., Ltd., Beijing 102600 (CN)
(72) Inventor: WANG, Haoyi, Beijing 102600 (CN); YE, Jianhua, Beijing 102600 (CN); GAO, Hui, Beijing 102600 (CN); DU, Xiaoming, Beijing 102600 (CN); WU, Yi, Beijing 102600 (CN); ZHOU, Peng, Beijing 102600 (CN); XIANG, Guanghai, Beijing 102600 (CN); WANG, Tianzi, Beijing 102600 (CN)
(74) Representative: LLR
(86) International application number: PCT/CN2021/116282
(87) International publication number: WO 2022/048604

(57) **Abstract**

The present application relates to a method for preparing a non-human mammal or progeny thereof, and the use thereof. The preparation method comprises the following steps: making a CH1 domain of an IgM heavy chain constant region not expressed or incorrectly expressed in the non-human mammal; and making one, two, three, four or more genes encoding an IgG heavy chain constant region in the non-human mammal not express or incorrectly express a CH1 domain during expression. The obtained non-human mammal or progeny thereof can be used for preparing a heavy chain antibody. The non-human mammal obtained by using the preparation method of the present application does not introduce any exogenous gene encoding heavy chain variable and constant regions of an antibody, and can directly use all VDJ genes encoding the heavy chain variable region of the antibody in its own genome, so as to produce more diverse heavy chain antibodies by means of the rearrangement of the heavy chain variable region.

## Description

### CROSS-REFERENCE

The present application claims priority to the invention patent application no. 202010924095.1, titled "METHOD FOR PREPARING NON-HUMAN MAMMAL OR PROGENY THEREOF, AND USE THEREOF" and filed on September 4, 2020, which is hereby incorporated by reference in its entirety.

### Technical Field

The present application relates to the field of biotechnology, in particular to a method for preparing a non-human mammal or progeny thereof, wherein the non-human mammal or progeny thereof can be used to produce a heavy chain antibody.

### Background Art

An antibody is a four-peptide-chain structure formed by connecting two identical heavy chains (H chains) and two identical light chains (L chains) by means of interchain non-covalent bonds or disulfide bonds. The heavy chain of antibody includes a heavy chain constant region (C_{H}) and a heavy chain variable region (V_{H}), wherein the heavy chain constant region of each of IgD, IgG and IgA includes four domains: CH1, a hinge region Hinge, CH2 and CH3; each of IgM and IgE includes fours domains: CH1, CH2, CH3 and CH4 (Janeway's Immunobiology, 9th Edition); the CH1 domain of the heavy chain constant region is connected to a light chain constant domain by means of a disulfide bond; the heavy chain variable region includes a hypervariable region of a complementary determining region (CDR) and a relatively conservative region called a framework region (FR); and the heavy chain variable region includes 3 CDRs and 4 FRs, which are arranged in the following order from an amino terminus to carboxy terminus: FR1, CDR1, FR2, CDR2, FR3, CDR3 and FR4.

According to differences in antigen specificity of the heavy chain constant regions of antibodies, the antibodies include the following five types: IgM, IgD, IgG, IgE and IgA. Since antibodies of the same class still have certain differences in antigen specificity in their heavy chain constant regions, they further can be divided into several isotypes, and for example, human IgG includes IgG1, IgG2, IgG3, IgG4, etc., and human IgA includes IgA1, IgA2, etc. The isotypes of antibodies of different mammals (such as humans, alpacas and mice) and of different varieties of mice (such as C57BL/6 mice and BALB/c mice) may differ.

A gene related to a heavy chain of an antibody includes five types of gene segments, i.e., L, V, D, J and C, wherein the heavy chain variable region is encoded by three types of gene segments, i.e., V, D and J, and the heavy chain constant region is encoded by the gene segment C. Antibody heavy chain genes of a human include about 40 V genes, 23 D genes, 6 J genes, and 9 C genes (source: Janeway's Immunobiology, 9th Edition, Page 177). A gene encoding an IgM heavy chain constant region is Ighm gene (Immunoglobulin heavy constant mu), a gene encoding an IgD heavy chain constant region is Ighd gene (Immunoglobulin heavy constant delta), a gene encoding an IgG heavy chain constant region is Ighg (Immunoglobulin heavy constant gamma), and a gene encoding an IgA heavy chain constant region is Igha (Immunoglobulin heavy constant alpha), etc. Correspondingly, a gene encoding an IgG1 heavy chain constant region is Ighg1 (Immunoglobulin heavy constant gamma 1), a gene encoding an IgG3 heavy chain constant region is Ighg3 (Immunoglobulin heavy constant gamma 3), and a gene encoding an IgG2b heavy chain constant region is Ighg2b (Immunoglobulin heavy constant gamma 2b). Diverse antibodies are prepared by means of the rearrangement of the heavy chain variable region. Mature B cells produce a secretory antibody IgM after being stimulated by an antigen, and second rearrangement occurs after antigen stimulation is performed again, and the class of immunoglobulin expressed and secreted on the membrane is switched from IgM with low affinity to IgG, IgA, IgE and other classes or isotypes of immunoglobulins. This phenomenon is called class switch or isotype switch.

A heavy chain antibody, also called a heavy chain only antibody, refers to an immunoglobulin antibody composed of only two heavy chains. Naturally occurring heavy chain antibodies are presented in camelids and sharks in the natural world. A heavy chain locus in a camelid germline contains a gene segment encoding a heavy chain constant region, and during maturation, and the rearranged VDJ-binding region is spliced to the 5' terminus of a gene segment encoding an IgG hinge region, so that the heavy chain constant region lacks a CH1 region that mediates binding to a light chain and cannot bind to the light chain, resulting in the preparation of IgG2 and IgG3 heavy chain antibodies. Heavy chain antibodies can also be produced by using genetically modified animals, and the produced heavy chain antibodies are also classified according to the difference in antigen specificity of the heavy chain constant regions of antibodies.

Compared with the molecular weight (150-160 kDa) of a traditional antibody, the heavy chain antibody is much smaller. One heavy chain of an IgG2c heavy chain antibody is about 40 kDa, and its heavy chain variable region that determines the antigen recognition specificity is only about 15 kDa. The heavy chain antibody is characterized by small molecular weight, can bind to some hidden epitopes, and are particularly suitable for targets of antibodies which are difficult to obtain.

The information disclosed in the background art is only intended to enhance the understanding of the general background of the present application, and should not be considered to recognize or imply in any form that the information constitutes the prior art that is well known to a person skilled in the art.

### Summary of the Invention

### Object of the invention

An object of the present application is to provide a method for preparing a non-human mammal or progeny thereof, and the use thereof.

### Solutions

In order to achieve the object of the present application, the present application provides the following technical solutions.

In a first aspect, the present application provides a method for preparing a non-human mammal or progeny thereof, wherein the method comprises the following steps:
making a CH1 domain of an IgM heavy chain constant region not expressed or incorrectly expressed in the non-human mammal; and
making one, two, three, four or more genes encoding an IgG heavy chain constant region in the non-human mammal not express or incorrectly express a CH1 domain during expression.

In a possible embodiment, according to the above-mentioned preparation method, making a CH1 domain of an IgM heavy chain constant region not expressed or incorrectly expressed in the non-human mammal is
only making a CH1 domain of an IgM heavy chain constant region not expressed or incorrectly expressed in the non-human mammal, or
making an IgM heavy chain constant region and an IgD heavy chain constant region not expressed or incorrectly expressed in the non-human mammal.

In a possible embodiment, according to the above-mentioned preparing method, making one, two, three, four or more genes encoding an IgG heavy chain constant region in the non-human mammal not express or incorrectly express a CH1 domain during expression is
making the first gene encoding the IgG heavy chain constant region in the non-human mammal not express or incorrectly express a CH1 domain during expression; or
making the first gene encoding the IgG heavy chain constant region in the non-human mammal not express or incorrectly express the IgG heavy chain constant region encoded thereby during expression and making one, two or three of the second or third or fourth genes encoding the IgG heavy chain constant region in the non-human mammal not express or incorrectly express a CH1 domain; or
making the first and second genes encoding the IgG heavy chain constant region in the non-human mammal not express or incorrectly express the IgG heavy chain constant region encoded thereby during expression and making one or two of the third or fourth genes encoding the IgG heavy chain constant region in the non-human mammal not express or incorrectly express a CH1 domain; or
making the first, second and third genes encoding the IgG heavy chain constant region in the non-human mammal not express or incorrectly express the IgG heavy chain constant region encoded thereby during expression and making the fourth gene encoding the IgG heavy chain constant region in the non-human mammal not express or incorrectly express a CH1 domain during expression; or
making the first gene encoding the IgG heavy chain constant region in the non-human mammal correctly express the IgG heavy chain constant region encoded thereby during expression and making one, two or three of the second or third or fourth genes encoding the IgG heavy chain constant region in the non-human mammal not express or incorrectly express a CH1 domain; or
making the first and second genes encoding the IgG heavy chain constant region in the non-human mammal correctly express the IgG heavy chain constant region encoded thereby during expression and making one or two of the third or fourth genes encoding the IgG heavy chain constant region in the non-human mammal not express or incorrectly express a CH1 domain; or
making the first, second and third genes encoding the IgG heavy chain constant region in the non-human mammal correctly express the IgG heavy chain constant region encoded thereby during expression and making the fourth gene encoding the IgG heavy chain constant region in the non-human mammal not express or incorrectly express a CH1 domain during expression. The present application further provides a non-human mammal in which a CH1 domain of an IgM heavy chain constant region is not expressed or incorrectly expressed and in which one, two, three, four or more genes encoding an IgG heavy chain constant region do not express or incorrectly express a CH1 domain.

In a possible embodiment, according to the above-mentioned non-human mammal, making a CH1 domain of an IgM heavy chain constant region not expressed or incorrectly expressed in the non-human mammal is
only making a CH1 domain of an IgM heavy chain constant region not expressed or incorrectly expressed; or
making an IgM heavy chain constant region and an IgD heavy chain constant region not expressed or incorrectly expressed.

In a possible embodiment, according to the above-mentioned non-human mammal, making one, two, three, four or more genes encoding an IgG heavy chain constant region in the non-human mammal not express or incorrectly express a CH1 domain is
making the first gene encoding the IgG heavy chain constant region not express or incorrectly express a CH1 domain; or
making the first gene encoding the IgG heavy chain constant region not express or incorrectly express the IgG heavy chain constant region encoded thereby, and making one, two or three of the second or third or fourth genes encoding the IgG heavy chain constant region not express or incorrectly express a CH1 domain; or
making the first and second genes encoding the IgG heavy chain constant region not express or incorrectly express the IgG heavy chain constant region encoded thereby, and making one or two of the third or fourth genes encoding the IgG heavy chain constant region not express or incorrectly express a CH1 domain; or
making the first, second and third genes encoding the IgG heavy chain constant region not express or incorrectly express the IgG heavy chain constant region encoded thereby, and making the fourth gene encoding the IgG heavy chain constant region not express or incorrectly express a CH1 domain; or
making the first gene encoding the IgG heavy chain constant region correctly express the IgG heavy chain constant region encoded thereby, and making one, two or three of the second or third or fourth genes encoding the IgG heavy chain constant region not express or incorrectly express a CH1 domain; or
making the first and second genes encoding the IgG heavy chain constant region correctly express the IgG heavy chain constant region encoded thereby, and making one or two of the third or fourth genes encoding the IgG heavy chain constant region not express or incorrectly express a CH1 domain; or
making the first, second and third genes encoding the IgG heavy chain constant region correctly express the IgG heavy chain constant region encoded thereby, and making the fourth gene encoding the IgG heavy chain constant region not express or incorrectly express a CH1 domain.

In a second aspect, the present application provides a method for preparing a non-human mammal or progeny thereof, wherein the method comprises the following steps:
knocking out a nucleotide sequence, comprising a nucleotide sequence encoding a CH1 domain of an IgM heavy chain constant region, on a genome of the non-human mammal; and
knocking out a target gene, comprising nucleotide sequence(s) encoding CH1 domain(s) on one, two, three, four or more genes encoding an IgG heavy chain constant region.

The present application further provides a non-human mammal, wherein a nucleotide sequence, comprising a nucleotide sequence encoding a CH1 domain of an IgM heavy chain constant region, and a target gene on a genome of the non-human mammal are knocked out. The target gene comprises:
nucleotide sequence(s) encoding CH1 domain(s) on one, two, three, four or more genes encoding an IgG heavy chain constant region.

In a possible embodiment, according to the above-mentioned preparation method or non-human mammal, knocking out a nucleotide sequence, comprising a nucleotide sequence encoding a CH1 domain of an IgM heavy chain constant region, on the genome of the non-human mammal is
only knocking out a nucleotide sequence encoding a CH1 domain of an IgM heavy chain constant region on a genome of the non-human mammal; or
knocking out nucleotide sequences, encoding an IgM heavy chain constant region and an IgD heavy chain constant region, on a genome of the non-human mammal.

In a possible embodiment, according to the above-mentioned preparation method or non-human mammal, the target gene is
a nucleotide sequence, encoding a CH1 domain, on the first gene encoding an IgG heavy chain constant region; or
all nucleotide sequences, encoding a CH1 domain, on a region starting from the first gene encoding the IgG heavy chain constant region to the second gene encoding the IgG heavy chain constant region; or
all nucleotide sequences, encoding a CH1 domain, on a region starting from the first gene encoding the IgG heavy chain constant region to the third gene encoding the IgG heavy chain constant region; or
all nucleotide sequences, encoding a CH1 domain, on a region starting from the first gene encoding the IgG heavy chain constant region to the fourth gene encoding the IgG heavy chain constant region; or
all nucleotide sequences, encoding a CH1 domain, on a region starting from the first gene encoding the IgG heavy chain constant region to the last gene encoding the IgG heavy chain constant region; or
a nucleotide sequence, encoding a CH1 domain, on the second gene encoding the IgG heavy chain constant region; or
all nucleotide sequences, encoding a CH1 domain, on a region starting from the second gene encoding the IgG heavy chain constant region to the third gene encoding the IgG heavy chain constant region; or
all nucleotide sequences, encoding a CH1 domain, on a region starting from the second gene encoding the IgG heavy chain constant region to the fourth gene encoding the IgG heavy chain constant region; or
a nucleotide sequence, encoding a CH1 domain, on the third gene encoding the IgG heavy chain constant region; or
all nucleotide sequences, encoding a CH1 domain, on a region starting from the third gene encoding the IgG heavy chain constant region to the fourth gene encoding the IgG heavy chain constant region; or
a nucleotide sequence, encoding a CH1 domain, on the fourth gene encoding the IgG heavy chain constant region.

In a possible embodiment, according to the above-mentioned preparation method, the following steps are completed in the same operation step or in different operation steps:
knocking out a nucleotide sequence, comprising a nucleotide sequence encoding a CH1 domain of an IgM heavy chain constant region, on a genome of the non-human mammal; and
knocking out a target gene.

In a possible embodiment, according to the above-mentioned preparation method or non-human mammal, the non-human mammal is a rodent; optionally, the rodent is a rat or a mouse; further optionally, the rodent is a mouse; and still further, the mouse is a C57BL/6 mouse or a BALB/c mouse.

In a possible embodiment, according to the above-mentioned preparation method or non-human mammal, the first gene encoding the IgG heavy chain constant region is Ighg3.

In a possible embodiment, according to the above-mentioned preparation method or non-human mammal, when the non-human mammal is a C57BL/6 mouse, the first gene encoding the IgG heavy chain constant region is Ighg3, the second gene encoding the IgG heavy chain constant region is Ighg1, the third gene encoding the IgG heavy chain constant region is Ighg2b, and the fourth gene encoding the IgG heavy chain constant region is Ighg2c; and
when the non-human mammal is a BALB/c mouse, the first gene encoding the IgG heavy chain constant region is Ighg3, the second gene encoding the IgG heavy chain constant region is Ighg1, the third gene encoding the IgG heavy chain constant region is Ighg2b, and the fourth gene encoding the IgG heavy chain constant region is Ighg2a.

In a possible embodiment, according to the above-mentioned preparation method or non-human mammal, the genome of the non-human mammal comprises a complete gene encoding a κ light chain and/or a λ light chain; and optionally, the κ light chain and/or the λ light chain can be normally expressed in the non-human mammal.

In a possible embodiment, according to the above-mentioned preparation method or non-human mammal, a gene knockout method comprises one or more of the followings: a gene targeting technology, a CRISPR/Cas9 method, a zinc finger nuclease method and a transcription activator-like effector nuclease method.

In a possible embodiment, according to the above-mentioned preparation method or non-human mammal, the non-human mammal or progeny thereof is used to produce a heavy chain antibody.

In a third aspect, the present application provides a method for preparing a C57BL/6 mouse or progeny thereof, wherein the method comprises the following steps:
knocking out genes, encoding an antibody IgM heavy chain constant region and an antibody IgD heavy chain constant region, in a genome of the C57BL/6 mouse; and
knocking out nucleotide sequences, encoding a CH1 domain, on a region starting from a gene encoding an IgG3 heavy chain constant region to a gene encoding an IgG2c heavy chain constant region in the genome of the C57BL/6 mouse.

The present application further provides a C57BL/6 mouse, wherein nucleotide sequences, which encode heavy chain constant regions of antibodies IgM, IgD, IgG1, IgG2b and IgG3, in a genome of the mouse and a nucleotide sequence, encoding a CH1 domain, on a gene encoding an antibody IgG2c heavy chain constant region, in the genome of the mouse are knocked out.

In a possible embodiment, according to the above-mentioned preparation method or C57BL/6 mouse, the genome of the C57BL/6 mouse comprises a complete gene encoding a κ light chain and/or an λ light chain; and optionally, the C57BL/6 mouse can normally express the κ light chain and/or the λ light chain.

In a possible embodiment, according to the above-mentioned preparation method or C57BL/6 mouse, a gene knockout method comprises one or more of the followings: a gene targeting technology, a CRISPR/Cas9 method, a zinc finger nuclease method and a transcription activator-like effector nuclease method.

In a possible embodiment, according to the above-mentioned preparation method or C57BL/6 mouse, all nucleotide sequences from the first exon of the gene encoding the IgM heavy chain constant region to the first exon of the gene encoding the IgG2c heavy chain constant region in the mouse are knocked out.

In a possible embodiment, according to the above-mentioned preparation method or C57BL/6 mouse, in the gene knockout step, sgRNA targeting the upstream portion of the first exon of the gene encoding the IgM heavy chain constant region in the mouse and sgRNA targeting the downstream portion of the first exon of the gene encoding the IgG2c heavy chain constant region in the mouse are a small a step.

In a possible embodiment, according to the above-mentioned preparation method or C57BL/6 mouse, a targeting sequence, which is targeted by sgRNA, on the upstream portion of the first exon of the gene encoding the IgM heavy chain constant region in the mouse comprises SEQ ID NO. 1 and SEQ ID NO. 2; and/or a targeting sequence, which is targeted by sgRNA, on the downstream portion of the first exon of the gene encoding the IgG2c heavy chain constant region in the mouse comprises SEQ ID NO. 3 and SEQ ID NO. 4.

In a possible embodiment, according to the above-mentioned preparation method or C57BL/6 mouse, the sgRNA targeting the upstream portion of the first exon of the gene encoding the IgM heavy chain constant region in the mouse is SEQ ID NO. 9 and SEQ ID NO. 10; and/or the sgRNA targeting the downstream portion of the first exon of the gene encoding the IgG2c heavy chain constant region in the mouse is SEQ ID NO. 11 and SEQ ID NO. 12.

In a possible embodiment, according to the above-mentioned preparation method or C57BL/6 mouse, the C57BL/6 mouse or progeny thereof is used to produce a heavy chain IgG2c antibody.

In a fourth aspect, the present application provides a method for preparing a C57BL/6 mouse or progeny thereof, wherein the method comprises the following steps:
knocking out a nucleotide sequence, encoding a CH1 domain, on a gene encoding an IgM heavy chain constant region in a genome of the C57BL/6 mouse; and
knocking out nucleotide sequences, encoding a CH1 domain, on a region starting from a gene encoding an IgG3 heavy chain constant region to a gene encoding an IgG2c heavy chain constant region in the genome of the C57BL/6 mouse.

The present application further provides a C57BL/6 mouse, a nucleotide sequence, which encodes the CH1 domain, on a gene encoding an IgM heavy chain constant region in a genome in the mouse and a nucleotide sequence, encoding a CH1 domain, on a region starting from a gene encoding an IgG3 heavy chain constant region to a gene encoding an IgG2c heavy chain constant region in a genome in the mouse are knocked out.

In a possible embodiment, according to the above-mentioned preparation method or C57BL/6 mouse, the genome of the C57BL/6 mouse comprises a complete gene encoding a κ light chain and/or an λ light chain; and optionally, the C57BL/6 mouse can normally express the κ light chain and/or the λ light chain.

In a possible embodiment, according to the above-mentioned preparation method or C57BL/6 mouse, a gene knockout method comprises one or more of the followings: a gene targeting technology, a CRISPR/Cas9 method, a zinc finger nuclease method and a transcription activator-like effector nuclease method.

In a possible embodiment, according to the above-mentioned preparation method or C57BL/6 mouse, in the gene knockout step, the first exon of the gene encoding the IgM heavy chain constant region in the mouse is knocked out, and all nucleotide sequences from the first exon of the gene encoding the IgG3 heavy chain constant region to the first exon of the gene encoding the IgG2c heavy chain constant region in the mouse are further knocked out.

In a possible embodiment, according to the above-mentioned preparation method or C57BL/6 mouse, in the gene knockout step, sgRNA targeting the upstream and downstream portions of the first exon of the gene encoding the IgM heavy chain constant region in the mouse is used, and sgRNA targeting the upstream portion of a first exon of a gene encoding an IgG3 heavy chain constant region in a mouse and sgRNA targeting the downstream portion of the first exon of the gene encoding IgG2c in the mouse are further used.

In a possible embodiment, according to the above-mentioned preparation method or C57BL/6 mouse, a targeting sequence, which is targeted by sgRNA, on the upstream portion of the first exon of the gene encoding the IgM heavy chain constant region in the mouse comprises SEQ ID NO. 1 and SEQ ID NO. 2; and/or
a targeting sequence, which is targeted by sgRNA, on the downstream portion of the first exon of the gene encoding the IgM heavy chain constant region in the mouse comprises SEQ ID NO. 5 and SEQ ID NO. 6; and/or
a targeting sequence, which is targeted by sgRNA, on the upstream portion of the first exon of the gene encoding the IgG3 heavy chain constant region in the mouse comprises SEQ ID NO. 7 and SEQ ID NO. 8; and/or
a targeting sequence, which is targeted by sgRNA, on the downstream portion of the first exon of the gene encoding the IgG2c heavy chain constant region in the mouse comprises SEQ ID NO. 3 and SEQ ID NO. 4.

In a possible embodiment, according to the above-mentioned preparation method or C57BL/6 mouse, the sgRNA targeting the upstream portion of the first exon of the gene encoding the IgM heavy chain constant region in the mouse is SEQ ID NO. 9 and SEQ ID NO. 10; and/or
the sgRNA targeting the downstream portion of the first exon of the gene encoding the IgM heavy chain constant region in the mouse is SEQ ID NO. 13 and SEQ ID NO. 14; and/or
the sgRNA targeting the upstream portion of the first exon of the gene encoding the IgG3 heavy chain constant region in the mouse is SEQ ID NO. 15 and SEQ ID NO. 16; and/or
the sgRNA targeting the downstream portion of the first exon of the gene encoding the IgG2c heavy chain constant region in the mouse is SEQ ID NO. 11 and SEQ ID NO. 12.

In a possible embodiment, according to the above-mentioned preparation method or C57BL/6 mouse, the C57BL/6 mouse or progeny thereof is used to produce a heavy chain IgG2c antibody.

In a fifth aspect, the present application provides a method for preparing a non-human mammal or progeny thereof, wherein the method comprises knocking out a nucleotide sequence, encoding a CH1 domain of an IgM heavy chain constant region, on a genome of the non-human mammal.

The present application further provides a non-human mammal, wherein a nucleotide sequence, encoding a CH1 domain of an IgM heavy chain constant region, on a genome of the non-human mammal is knocked out.

In a possible embodiment, according to the above-mentioned preparation method or non-human mammal, the non-human mammal is a rodent; optionally, the rodent is a rat or a mouse; further optionally, the rodent is a mouse; and still further, the mouse is a C57BL/6 mouse or a BALB/c mouse.

In a possible embodiment, according to the above-mentioned preparation method or non-human mammal, the non-human mammal or progeny thereof is used to construct the above-mentioned non-human mammal or progeny thereof.

In a sixth aspect, the present application provides the use of a non-human mammal or offspring thereof constructed by using the above-mentioned preparation method, a C57BL/6 mouse or offspring thereof constructed by using the above-mentioned preparation method, the above-mentioned non-human mammal and the above-mentioned C57BL/6 mouse in the screening of a target heavy chain antibody.

In a possible embodiment, according to the use, a phage display method is used when the target heavy chain antibody is screened.

In a possible embodiment, according to the use, the screening of the target heavy chain antibody is the screening of a C-reactive coronavirus S protein or coronavirus N protein antigen-specific IgG2c heavy chain antibody.

In a seventh aspect, the present application provides a method for screening a target heavy chain antibody, wherein the method comprises performing screening by using a non-human mammal or progeny thereof constructed by using the preparation method derived from the above-mentioned first aspect, a non-human mammal or progeny thereof constructed by using the preparation method derived from the above-mentioned the second aspect, a C57BL/6 mouse or progeny thereof constructed by the preparation method derived from the above-mentioned third aspect, or a C57BL/6 mouse or progeny thereof constructed by using the preparation method derived from the above-mentioned fourth aspect as an immune animal.

In a possible embodiment, according to the above-mentioned method, a phage display method is used when the target heavy chain antibody is screened.

In a possible embodiment, according to the method, the screening of the target heavy chain antibody is the screening of a C-reactive coronavirus S protein or coronavirus N protein antigen-specific IgG2c heavy chain antibody.

In an eighth aspect, the present application provides a non-human mammal cell or cell line or primary cell culture, wherein the non-human mammal cell or cell line or primary cell culture is derived from a non-human mammal or progeny thereof constructed by using the above-mentioned preparation method, or the C57BL/6 mouse or progeny thereof constructed by using the above-mentioned preparation method.

In a ninth aspect, the present application provides an in-vitro tissue or in-vitro organ or a culture thereof, the in-vitro tissue or in-vitro organ or the culture thereof is derived from the non-human mammal or progeny thereof constructed by using the above-mentioned preparation method, or the C57BL/6 mouse or progeny thereof constructed by using the above-mentioned preparation method.

In the tenth aspect, the present application provides an sgRNA composition, wherein the sgRNA composition comprises: sgRNA targeting the upstream portion of the first exon of the gene encoding the IgM heavy chain constant region in the mouse and sgRNA targeting the downstream portion of the first exon of the gene encoding the IgG2c heavy chain constant region in the mouse;
sgRNA targeting the upstream and downstream portions of a first exon of a gene encoding an IgM heavy chain constant region in a mouse; or
sgRNA targeting the upstream portion of a first exon of a gene encoding an IgG3 heavy chain constant region in a mouse and sgRNA targeting the downstream portion of the first exon of the gene encoding IgG2c in the mouse.

In a possible embodiment, according to the sgRNA composition, a targeting sequence, which is targeted by sgRNA, on the upstream portion of the first exon of the gene encoding the IgM heavy chain constant region in the mouse comprises SEQ ID NO. 1 and SEQ ID NO. 2; and/or
a targeting sequence, which is targeted by sgRNA, on the downstream portion of the first exon of the gene encoding the IgG2c heavy chain constant region in the mouse comprises SEQ ID NO. 3 and SEQ ID NO. 4; and/or
a targeting sequence, which is targeted by sgRNA, on the downstream portion of the first exon of the gene encoding the IgM heavy chain constant region in the mouse comprises SEQ ID NO. 5 and SEQ ID NO. 6; and/or
a targeting sequence, which is targeted by sgRNA, on the upstream portion of the first exon of the gene encoding the IgG3 heavy chain constant region in the mouse comprises SEQ ID NO. 7 and SEQ ID NO. 8.

In a possible embodiment, according to the sgRNA composition, the sgRNA targeting the upstream portion of the first exon of the gene encoding the IgM heavy chain constant region in the mouse is SEQ ID NO. 9 and SEQ ID NO. 10; and/or
the sgRNA targeting the downstream portion of the first exon of the gene encoding the IgM heavy chain constant region in the mouse is SEQ ID NO. 13 and SEQ ID NO. 14; and/or
the sgRNA targeting the upstream portion of the first exon of the gene encoding the IgG3 heavy chain constant region in the mouse is SEQ ID NO. 15 and SEQ ID NO. 16; and/or
the sgRNA targeting the downstream portion of the first exon of the gene encoding the IgG2c heavy chain constant region in the mouse is SEQ ID NO. 11 and SEQ ID NO. 12.

In an eleventh aspect, the present application provides a knockout vector. The knockout vector comprises one or more segments of DNA sequences encoding sgRNA, wherein the targeting sequence targeted by the sgRNA is selected from one of the followings:
a targeting sequence on the upstream portion of a first exon of a gene encoding an IgM heavy chain constant region in a mouse; or
a targeting sequence on the downstream portion of a first exon of a gene encoding an IgG2c heavy chain constant region in a mouse; or
a targeting sequence on the downstream portion of a first exon of a gene encoding an IgM heavy chain constant region in a mouse; or
a targeting sequence on the upstream portion of a first exon of a gene encoding an IgG3 heavy chain constant region in a mouse.

In a possible embodiment, according to the above-mentioned knockout vector, a backbone of the knockout vector is an sgRNA expression vector.

In a twelfth aspect, the present application provides a cell containing the above-mentioned knockout vector.

Incorrect expression: incorrect expression is opposite to correct expression, and both correct expression and incorrect expression in the present application actually have meanings commonly understood in the art. For example, incorrectly expressing a CH1 domain refers to that mutation or deletion at the genomic level causes incorrect expression of a CH1 domain of a heavy chain constant region, which leads to the CH1 domain losses the ability to bind to a light chain; and for example, mutation or deletion occurs in a range of 10 nucleotides on each of the upstream and downstream portion of an exon encoding a CH1 domain of a heavy chain constant region, which leads to the CH1 domain losses the ability to bind to a light chain. For another example, a gene encoding an IgG heavy chain constant region incorrectly expresses the IgG heavy chain constant region encoded thereby during expression, which means that the immunoglobulin antibody expressed by the gene does not have antibody effects; and by contrast, a gene encoding an IgG heavy chain constant region correctly expresses the encoded IgG heavy chain constant region during expression, which means that the immunoglobulin antibody expressed by the gene has antibody effects;
the first gene encoding the IgG heavy chain constant region, the second gene encoding the IgG heavy chain constant region, the third gene encoding the IgG heavy chain constant region, and the fourth gene encoding the IgG heavy chain constant region refer to that on a gene locus encoding the IgG heavy chain constant region, genes encoding the IgG heavy chain constant region are arranged in a sequence from the upstream portion to the downstream portion.

### Beneficial effects

In the present application, by means of making a CH1 domain of an IgM heavy chain constant region not expressed or incorrectly expressed in a non-human mammal and making one or more genes encoding an IgG heavy chain constant region not express or incorrectly express a CH1 domain during expression, the non-human mammal or progeny thereof is prepared and can be used for preparing a heavy chain antibody. The non-human mammal obtained by using the preparation method of the present application does not introduce any exogenous gene encoding heavy chain variable and constant regions of an antibody, and can directly use all VDJ genes encoding the heavy chain variable region of the antibody in its own genome, so as to produce more diverse heavy chain antibodies by means of the rearrangement of the heavy chain variable region.

In immunology, it is generally believed that IgM is extremely important for the growth of B cells, whereas the growth of B cells is also extremely important for the preparation of the antibody. In the present application, it is verified through experiments that even if a CH1 domain of an IgM heavy chain constant region is not expressed and encoded or even a gene encoding an IgM heavy chain constant region and a gene encoding an IgD heavy chain constant region are deleted, the immunologic maturation of a non-human mammal is not significantly affected, all mice survive normally, high-titer immune response still can be produced, and a heavy chain antibody with strong specificity and high affinity can be obtained therefrom by means of screening.

By selecting a genetically modified gene encoding an IgG heavy chain constant region, a specific isotype of heavy chain IgG antibody can be obtained.

### Brief Description of the Drawings

One or more examples are illustrated by means of figures in the drawings corresponding to the examples, and these illustrations are not intended to limit the examples. The word "exemplary" used exclusively herein means "serving as an example, embodiment or illustration". Any embodiment illustrated herein as "exemplary" need not be construed as being superior or better than other embodiments.

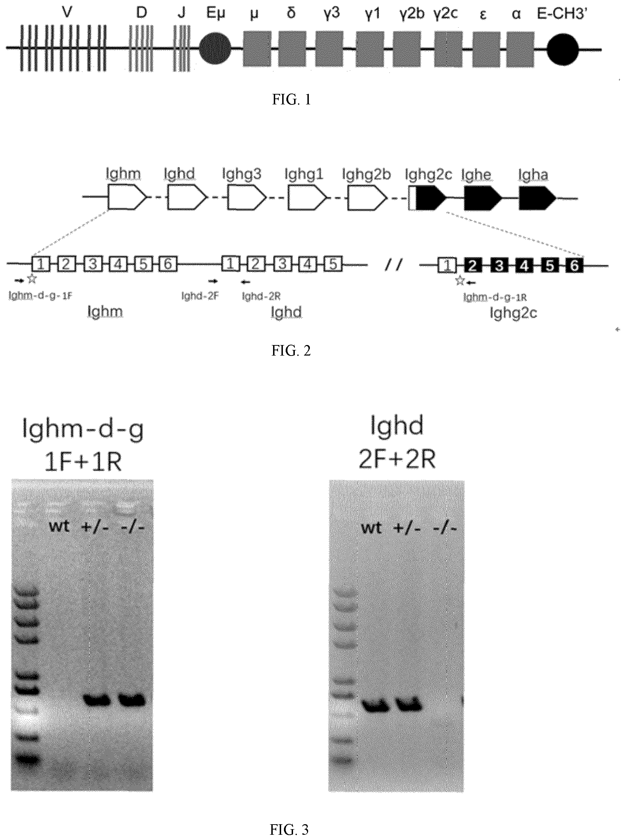

FIG. 1 is a schematic diagram of genetic loci of antibody heavy chain variable and constant regions of a mouse involved in example 1 of the present application.
FIG. 2 is a schematic diagram of an sgRNA targeting strategy for the preparation of an Ighm-d-g mouse in example 1 of the present application, wherein boxes in the second row represent exons, the white boxes represent gene segments that are knocked out, and numbers are serial numbers of the exons; the five-star represents target positions for sgRNA; and arrows represent positions targeted by a primer for genotype identification.
FIG. 3 show schematic diagrams of PCR glue diagram for genotype identification of Ighm-d-g mice in example 1 of the present application, wherein wt: wild type; +/-: heterozygote; -/-: knock out homozygote.

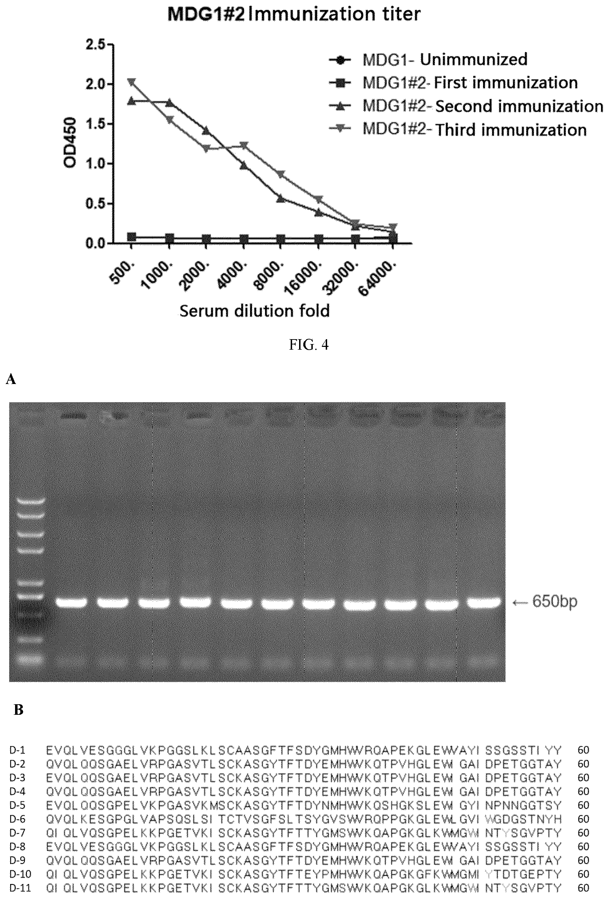

FIG. 4 is a schematic diagram of the detection result of serum titers obtained after an Ighm-d-g homozygous mouse is immunized with CRP (a human C-reactive protein) as an antigen protein in example 2 of the present application, wherein the serum titers are both very low after the first immunization and when the mouse is unimmunized, and the two lines are overlapped.

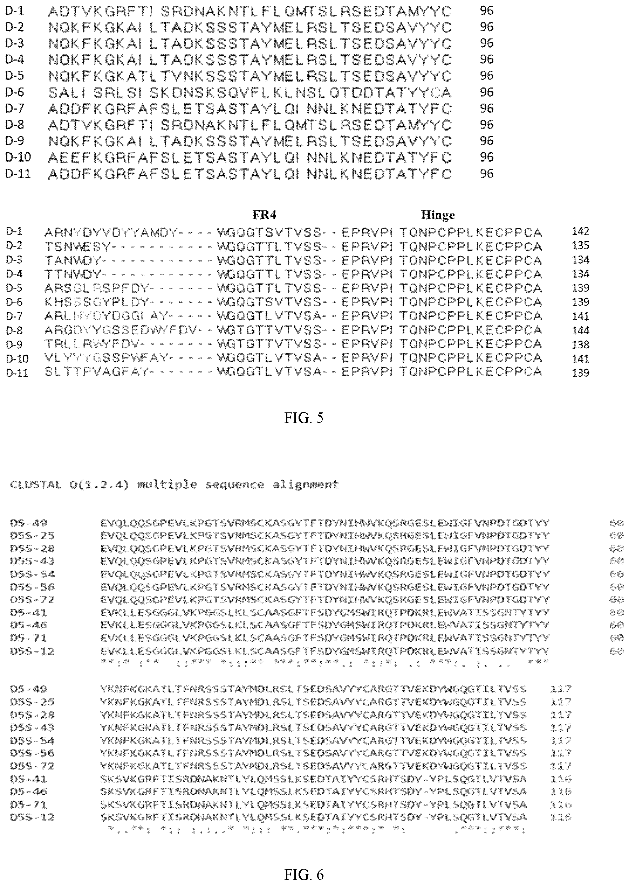

FIG. 5A is a colony PCR diagram of a VH fragment of a heavy chain gene in an IgG2c antibody of an Ighm-d-g homozygous mouse in example 5 of the present application; and FIG. 5B is a comparison diagram of heavy chain amino acid sequences of IgG2c antibodies expressed by Ighm-d-g homozygous mice in example 5 of the present application.
FIG. 6 shows sequence alignment of positive clones of the heavy chain antibody phage library screening in example 6 of the present application.

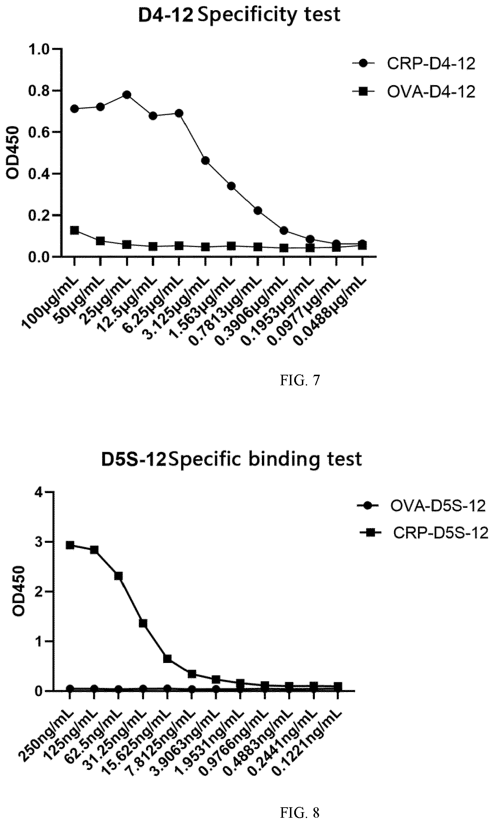

FIG. 7 shows ELISA results of specific recognition of purified D4-12 to antigen CRP in example 7 of the present application.
FIG. 8 shows ELISA results of specific recognition of purified 5S-12 to antigen CRP in example 8 of the present application, wherein the OD450 values of two lines OVA-D5S-12 and BSA-D5S-12 are very low, and the lines are overlapped.

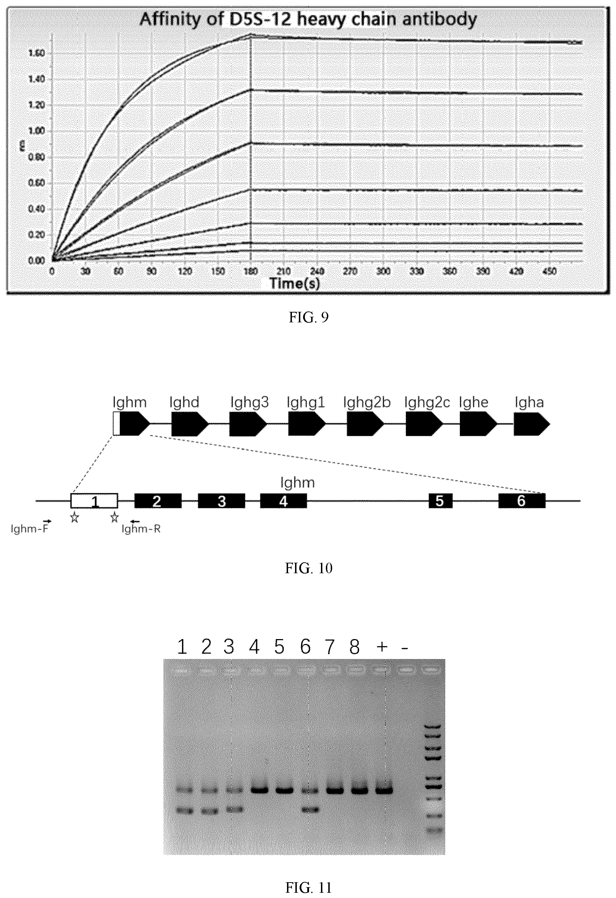

FIG. 9 shows affinity determination result of a D5S-12 heavy chain antibody in example 8 of the present application.
FIG. 10 is a schematic diagram of an sgRNA targeting strategy for preparing an IghM mouse in example 9 of the present application, wherein black boxes represent exons, and numbers are serial numbers of exons; the five star symbol represents target positions for sgRNA; and arrows represent primers for genotype identification.
FIG. 11 shows an example of PCR identification of an IghM mouse genotype in example 9 of the present application, wherein the Ighm wild-type genes produce a band of about 600 bp, and the Ighm genes which are knocked out produce a band of about 300 bp; and numbers represent serial numbers of mouse samples, +: wild-type control, -: blank control.

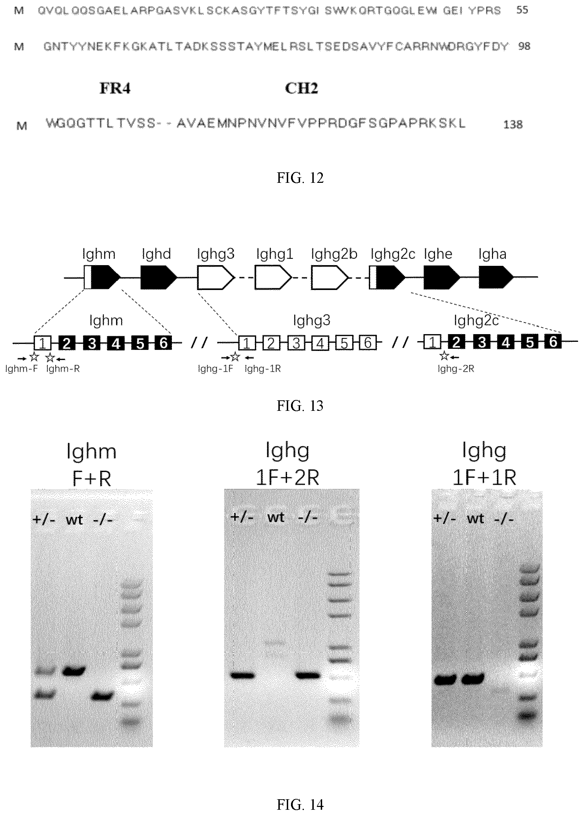

FIG. 12 is a diagram of an amino acid sequence of a heavy chain of an IgM antibody expressed by an IghM homozygous mouse in example 10 of the present application.
FIG. 13 is a schematic diagram of an sgRNA targeting strategy for the preparation of an IghM-3G3 mouse in example 11 of the present application, wherein black boxes represent exons, and numbers are serial numbers of exons; the five-star symbol represents target positions for sgRNA; and arrows represent primers for genotype identification.
FIG. 14 show schematic diagrams of PCR glue diagram for genotype identification of IghM-3G3 mice in example 11 of the present application, wherein wt: wild type; +/-: heterozygote; -/-: knock out homozygote.

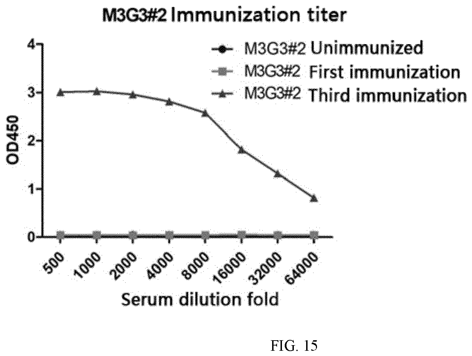

FIG. 15 is a schematic diagram of detection results of serum titers obtained after IghM-3G3 homozygous mice are immunized with CRP (a human C-reactive protein) as an antigen protein in example 12 of the present application, wherein the serum titers are both very low after the first immunization and the second immunization, and the two lines are overlapped.

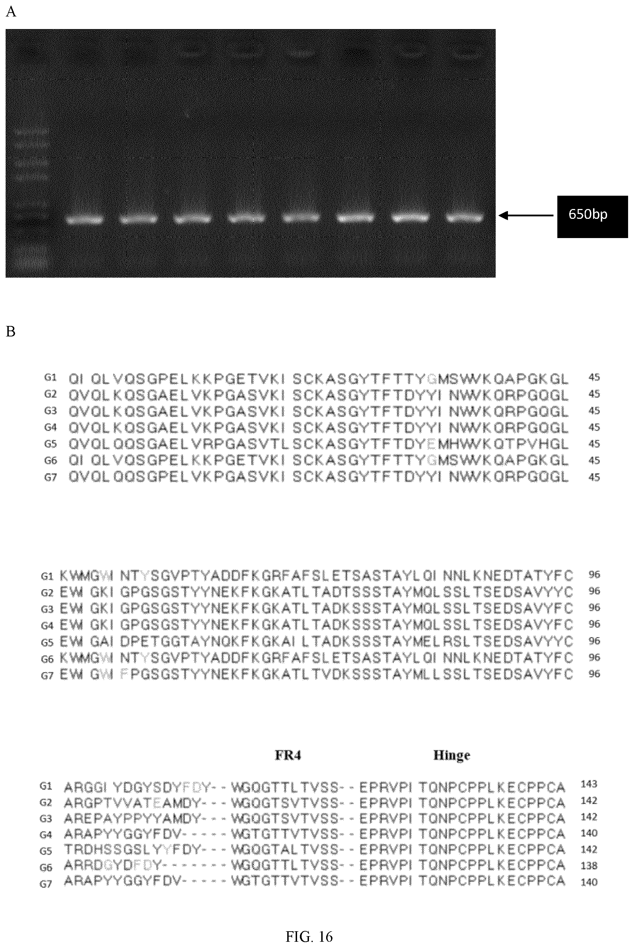

FIG. 16A is a colony PCR map of a VH fragment of a heavy chain gene in an IgG2c antibody of an IghM-3G3 homozygous mouse in example 13 of the present application; and FIG. 16B is a comparison diagram of heavy chain amino acid sequences of IgG2c antibodies expressed by IghM-3G3 homozygous mice in example 13 of the present application.

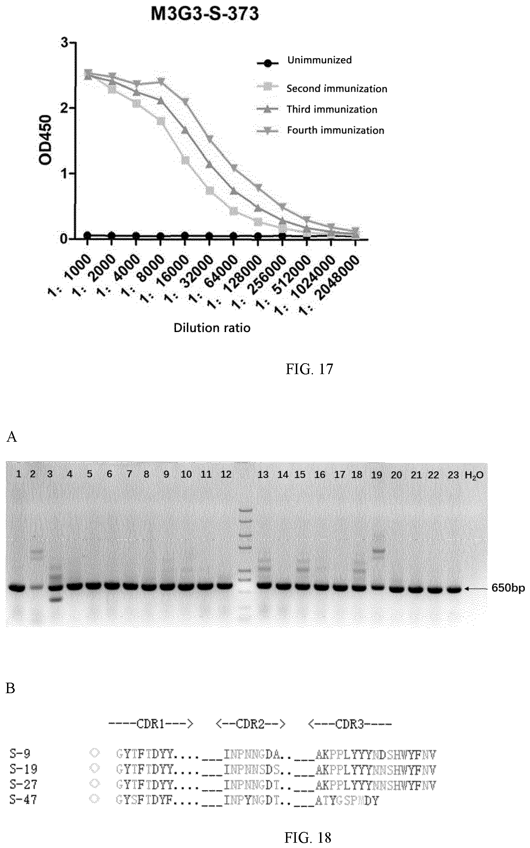

FIG. 17 is a diagram showing results of serum titers detected by ELISA as mentioned in example 15 of the present application, wherein in the case that an IghM-3G3 homozygous mouse is immunized with a coronavirus S protein antigen, blood samples are collected when the mouse is unimmunized, one week after the second immunization, one week after the third immunization and one week after the fourth immunization, respectively, and the serum is subjected to doubling dilution by using PBS.

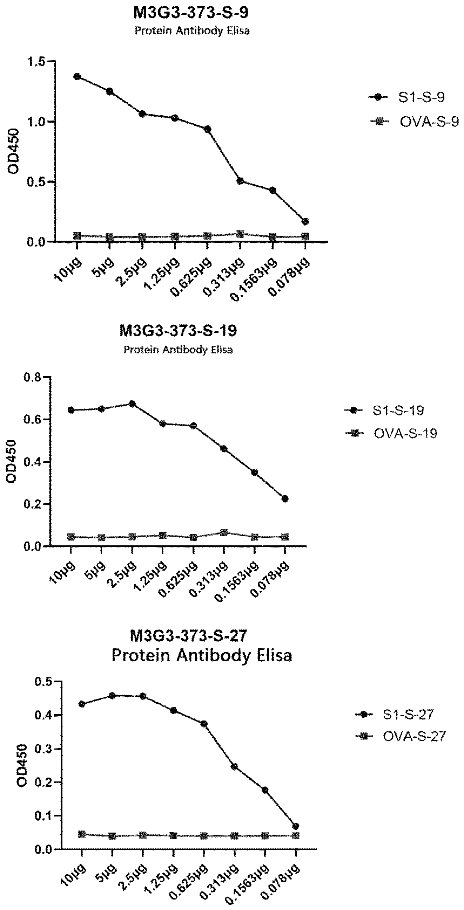

FIG. 18A is a diagram showing colony PCR identification of an IgG2c antibody heavy chain gene in an IghM-3G3 homozygous mouse immunized with a coronavirus S protein antigen as mentioned in example 16 of the present application; and FIG. 18B is a comparison diagram showing CDR regions amino acid sequence of positive clones obtained by phage display library construction and antibody panning for an IgG2c antibody heavy chain gene of an IghM-3G3 homozygous mouse immunized with a coronavirus S protein antigen, as mentioned in example 17 of the present application.

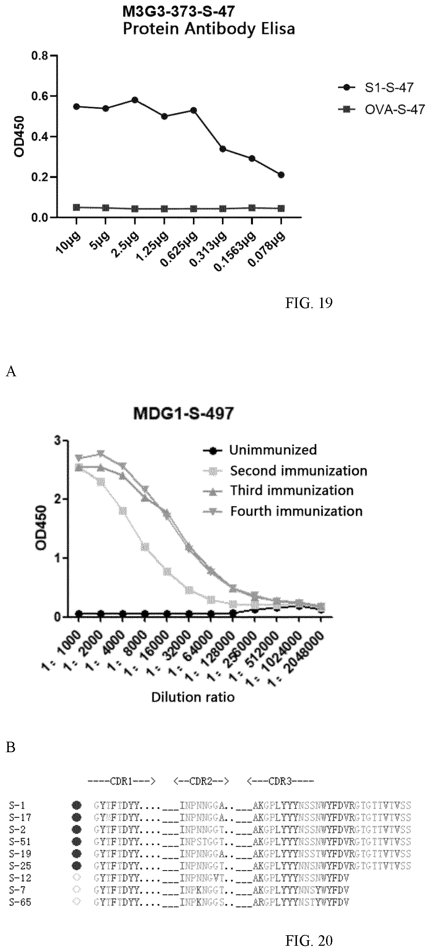

FIG. 19 is a diagram showing results of detecting whether antibodies S-9, S-19, S-27 and S-47 can specifically recognize and bind to an antigen coronavirus S protein by means of ELISA as mentioned in example 17 of the present application.

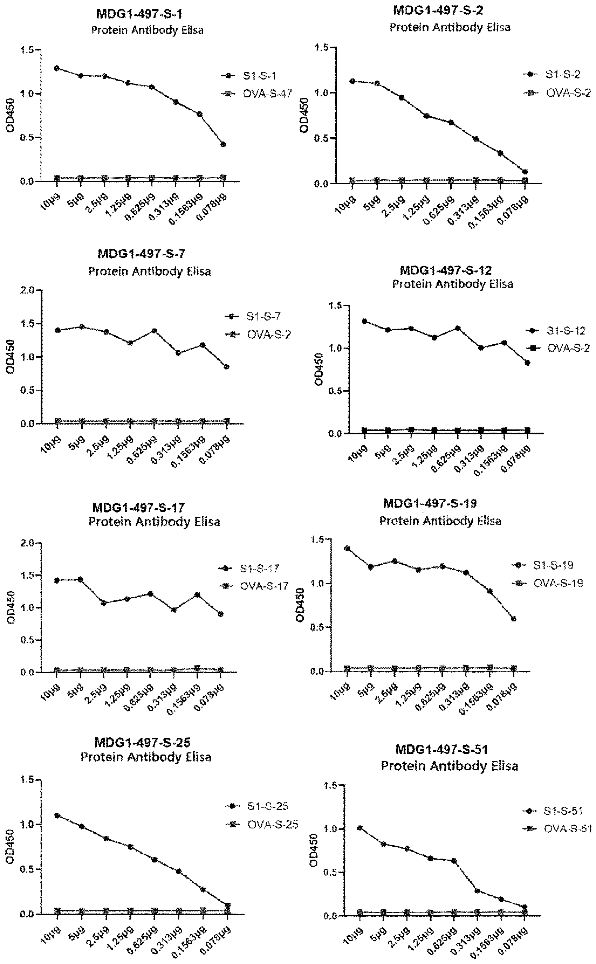

FIG. 20A is a diagram showing results of serum titers detected by ELISA in example 18 the present application, wherein in the case that an IghM-DG1 homozygous mouse is immunized with a coronavirus S protein antigen, blood samples are collected when the mouse is unimmunized, one week after the second immunization, one week after the third immunization and one week after the fourth immunization, respectively, and the serum is subjected to doubling dilution by using PBS; and FIG. 20B is a diagram showing amino acid sequence alignment of CDR regions of positive clones obtained by phage display library construction and antibody panning for an IghM-DG1 homozygous mouse immunized with a coronavirus S protein antigen as described above.

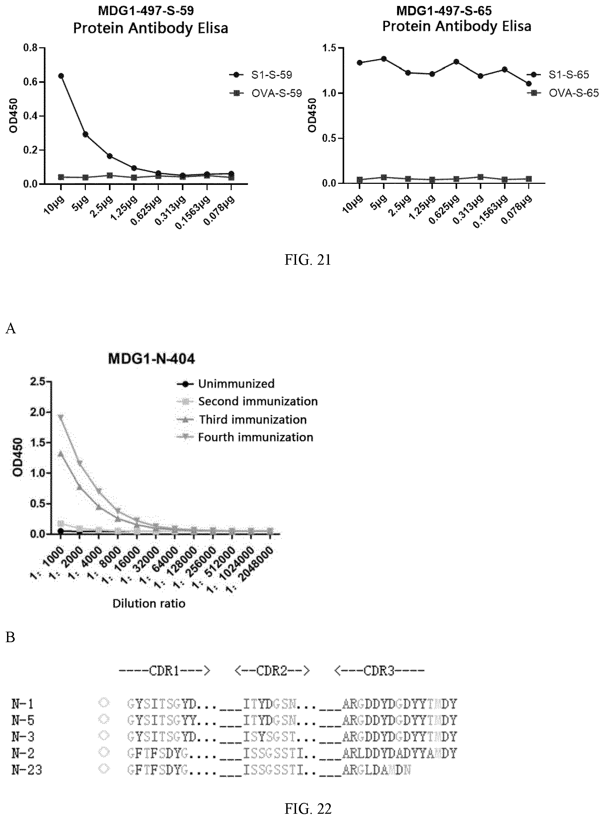

FIG. 21 is a diagram showing results of detecting whether antibodies S-1, S-7, S-12, S-17, S19, S-25, S-51 and S-65 can specifically recognize and bind to an antigen coronavirus S protein by means of ELISA as mentioned in example 18 of the present application.

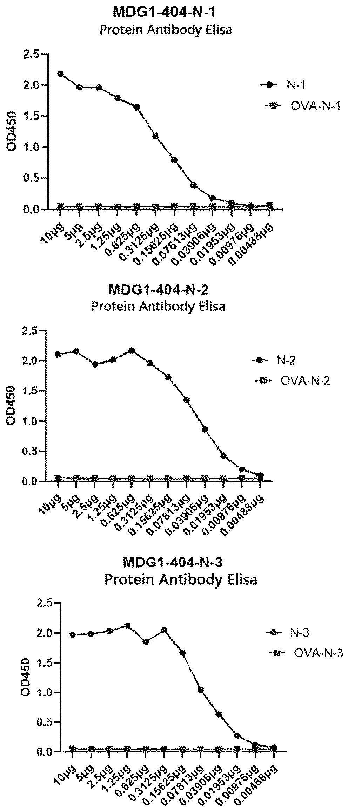

FIG. 22A is a diagram showing results of serum titers detected by ELISA as mentioned in example 19 of the present application, wherein in the case that an IghM-DG1 homozygous mouse is immunized with a coronavirus N protein antigen, blood samples are collected when the mouse is unimmunized, one week after the second immunization, one week after the third immunization and one week after the fourth immunization, respectively, and the serum is subjected to doubling dilution by using PBS; and FIG. 22B is a diagram showing amino acid sequence alignment of CDR regions of positive clones obtained by phage display library construction and antibody panning for an IghM-DG1 homozygous mouse immunized with a coronavirus N protein antigen as described above.

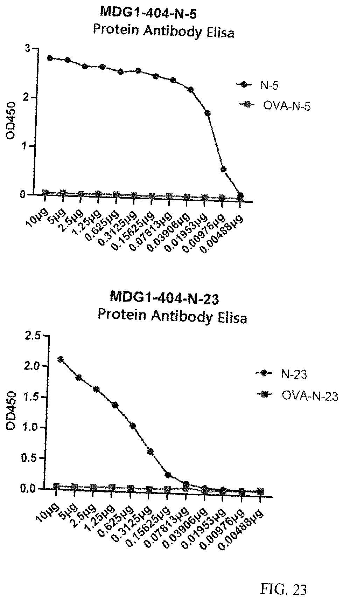

FIG. 23 is a diagram showing results of detecting whether antibodies N-1, N-2, N-3, N-5 and N-23 can specifically recognize and bind to an antigen coronavirus S protein by means of ELISA as mentioned in example 19 of the present application.

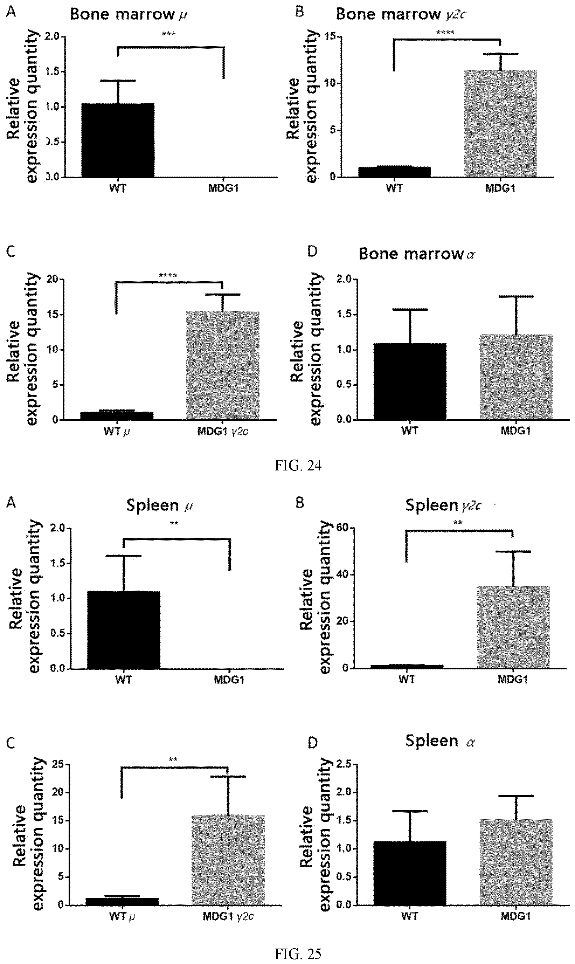

FIG. 24 shows comparison of relative expression quantities of immunoglobulin genes in bone marrow of an MDG1 mouse and a wild-type mouse mentioned in example 20 of the present application. FIGs. 24A-24D show relative expression quantities of a µ gene, a y2c gene and a y2c gene in bone marrow of mice with two genotypes in comparison with a µ gene and an α gene, respectively, wherein MDG1 is a homozygous knockout mouse, WT is a wild-type mouse, BM is bone marrow, the housekeeping gene Gapdh is used as an internal reference, n = 4, data analysis is performed by using a calculation method of 2^{-ΔΔCt}, statistical analysis is performed by using a two-tailed T test, and ^{∗∗∗} represents p < 0.01.
FIG. 25 shows comparison of relative expression quantities of immunoglobulin genes in spleens of an MDG1 mouse and a wild-type mouse mentioned in example 20 of the present application. FIGs. 25A-25D show relative expression quantities of a µ gene, a y2c gene and a y2c gene in spleens of mice with two genotypes in comparison with a µ gene and an α gene, respectively, wherein MDG1 is a homozygous knockout mouse, WT is a wild-type mouse bone marrow, the housekeeping gene Gapdh is used as an internal reference, n = 4, data analysis is performed by using a calculation method of 2^{-ΔΔCt}, statistical analysis is performed by using a two-tailed T test, and ** represents p < 0.01.

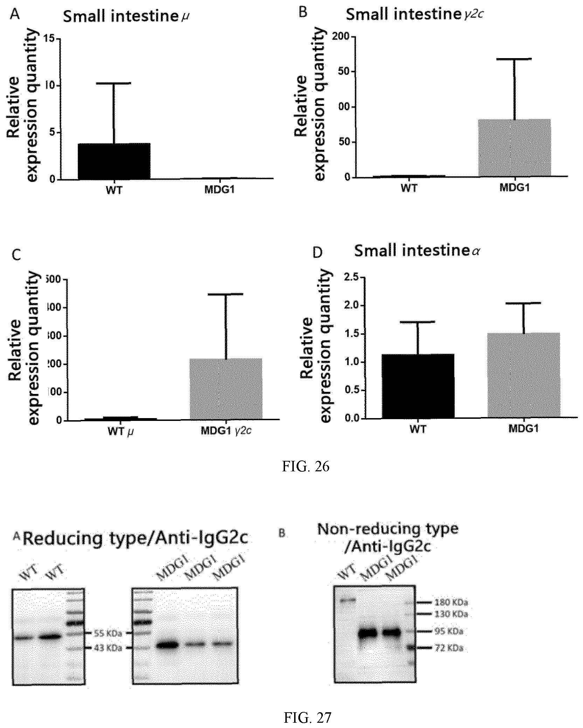

FIG. 26 shows comparison of relative expression quantities of immunoglobulin genes in small intestines of an MDG1 mouse and a wild-type mouse mentioned in example 20 of the present application. FIGs. 26A-26D show relative expression quantities of a µ gene, a y2c gene and a y2c gene in small intestines of mice with two genotypes in comparison with a µ gene and an α gene, respectively, wherein MDG1 is a homozygous knockout mouse, WT is a wild-type mouse, SI is the small intestine, the housekeeping gene Gapdh is used as an internal reference, n = 4, data analysis is performed by using a calculation method of 2^{-ΔΔCt}, and statistical analysis is performed by using a two-tailed T test.
FIG. 27 shows an expression form of IgG2c in serum of an MDG1 mouse as detected by Western Blot as mentioned in example 21 of the present application, wherein WT represents a wild-type mouse and MDG1 represents a homozygous knockout mouse. FIG. 27A shows detection of an expression form of IgG2c under reducing conditions; and FIG. 27B shows detection of an expression form of IgG2c under non-reducing conditions, wherein the serum of a wild-type mouse is subjected to 20-fold dilution, and the serum of MDG1 mice is subjected to 100-fold dilution, and Goat Anti-Mouse IgG2c heavy chain (HRP) is subjected to 10000-fold dilution.

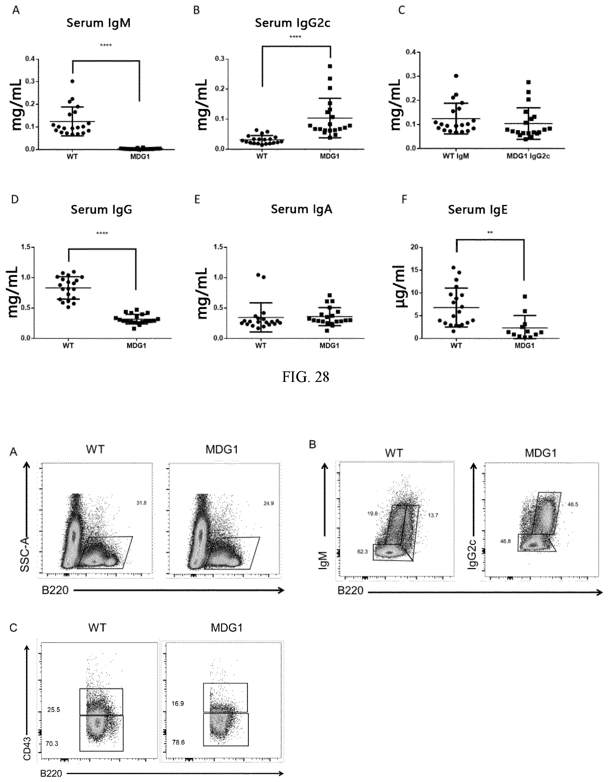

FIG. 28 shows comparison of the expression quantities of immunoglobulin in serum of an MDG1 mouse and a wild-type mouse under a quiescent condition as mentioned in example 22 of the present application. FIGs. 28A-28F show the expression quantities of IgM and IgG2c in serum of mice with two genotypes, IgM of a wild-type mouse and IgG2c of an MDG1 mouse, total IgG, IgA and IgE, respectively, wherein n = 20, statistical analysis is performed by using a two-tailed T test, ^{∗} represents p < 0.05 and ^{∗∗∗∗} represents p < 0.01.

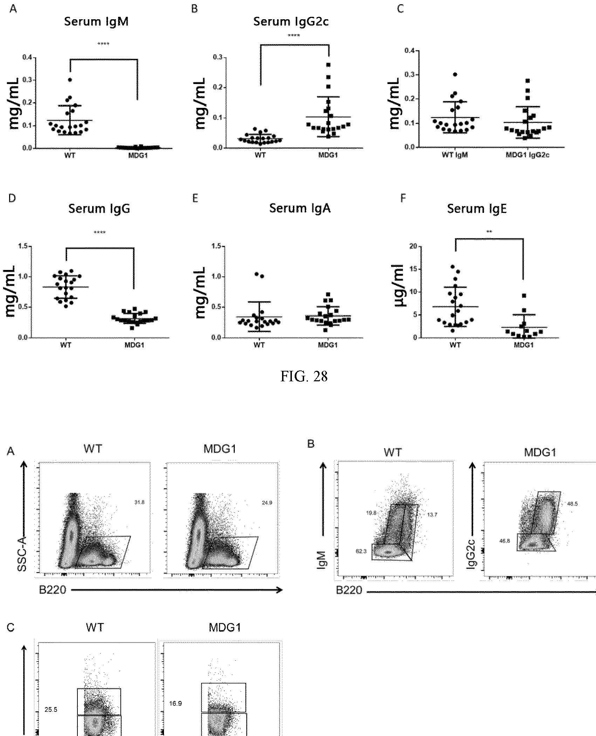

FIG. 29 shows the growth states of B cells in the bone marrow of an MDG1 mouse mentioned in example 23 of the present application. FIGs. 29A-29C show the grouping states of bone marrow B cells, immature B cells/mature B cells, pro-B cells/pre-B cells, and FIGs. 29D-29F show the proportions and numbers of bone marrow B cells, pro-B cells and pre-B cells, respectively, wherein WT is a wild-type mouse, MDG1 is a homozygous knockout mice, pro-B cells are progenitor B cells, and pre-B cells are precursor B cells; and n = 6, statistical analysis is performed by using a two-tailed T test, ^{∗} represents p < 0.05 and ^{∗∗∗} represents p < 0.01.

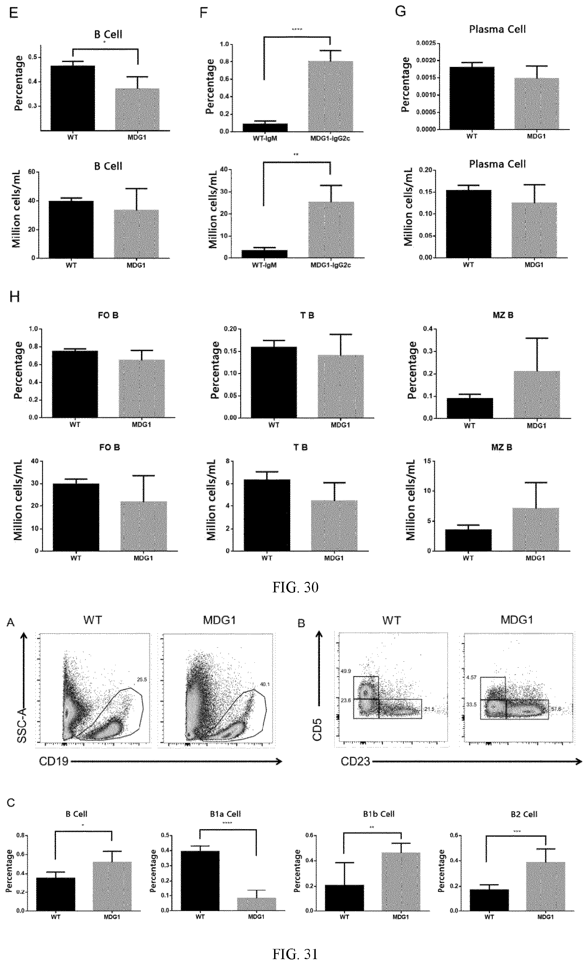

FIG. 30 shows the growth states of B cells in the spleen of an MDG1 mouse mentioned in example 23 of the present application. FIGs. 30A-30D show the grouping states of spleen B cells, IgG2c + /IgM + B cells, follicular B cells/marginal zone B cells/transitional B cells and plasma cells, and FIGs. 30E-30H show the proportions and numbers of spleen B cells, IgG2c + /IgM + B cells, plasma cells, and follicular B cells/marginal zone B cells/transitional B cells, wherein WT is a wild-type mouse, MDG1 is a homozygous knockout mouse, FO B is a follicular B cell, T B is a transitional B cell, MZ B is a marginal zone B cell, plasma is a plasma cell, n = 4, statistical analysis is performed by using a two-tailed T test, ^{∗} represents p < 0.05, and ^{∗∗} represents p < 0.01.
FIG. 31 shows the growth states of B cells in the peritoneal cavity of an MDG1 mouse mentioned in example 23 of the present application. FIGs. 31A and 31B show the grouping states of peritoneal cavity B cells and B1a/B1b/B2 cells, respectively; and FIG. 31C shows the proportions of peritoneal cavity B cells, B1a cells, B1b cells and B2 cells, respectively, wherein WT is a wild-type mouse and MDG1 is a homozygous knockout mouse; and n = 7, statistical analysis is performed by using a two-tailed T test, * represents p < 0.05 and ** represents p < 0.01.

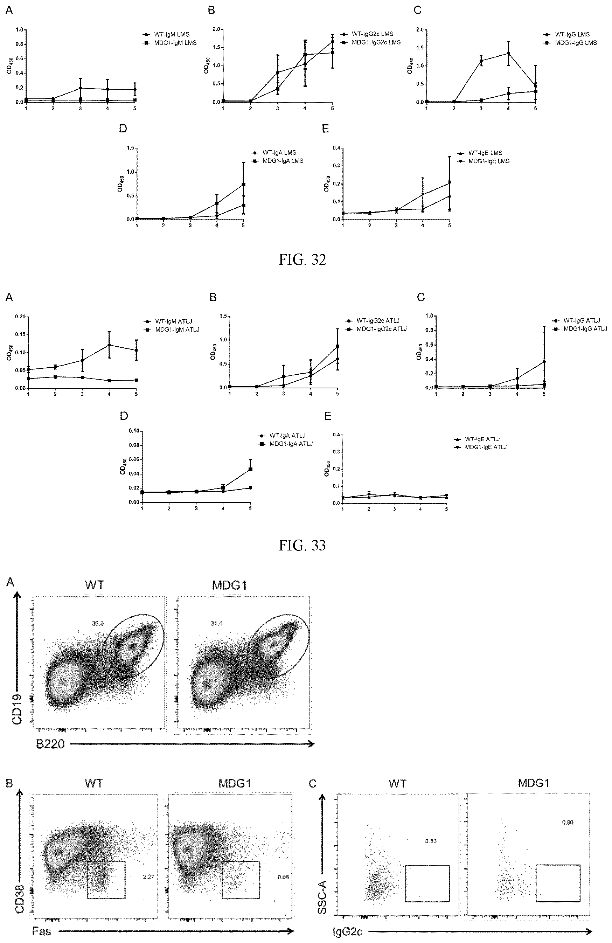

FIG. 32 shows change trends of relative expression quantities of antigen-specific antibodies in the serum of a wild-type mouse and an MDG1 mouse after chloramphenicol immunization as mentioned in example 25 of the present application. FIGs. 32A-32E show relative expression quantities of chloramphenicol-specific IgM, IgG2c, IgG, IgA and IgE, respectively, wherein the abscissa represents the number of times of immunizations, LMS represents chloramphenicol, MDG1 is a homozygous knockout mouse, WT is a wild-type mouse, and n = 7.
FIG. 33 shows change trends of relative expression quantities of antigen-specific antibodies in the serum of a wild-type mouse and an MDG1 mouse after atrazine immunization as mentioned in example 25 of the present application. FIGs. 33A-33E show relative expression quantities of atrazine-specific IgM, IgG2c, IgG, IgA and IgE, respectively, wherein the abscissa represents the number of times of immunizations, ATLJ represents atrazine, MDG1 is a homozygous knockout mouse, WT is a wild-type mouse, and n = 3.

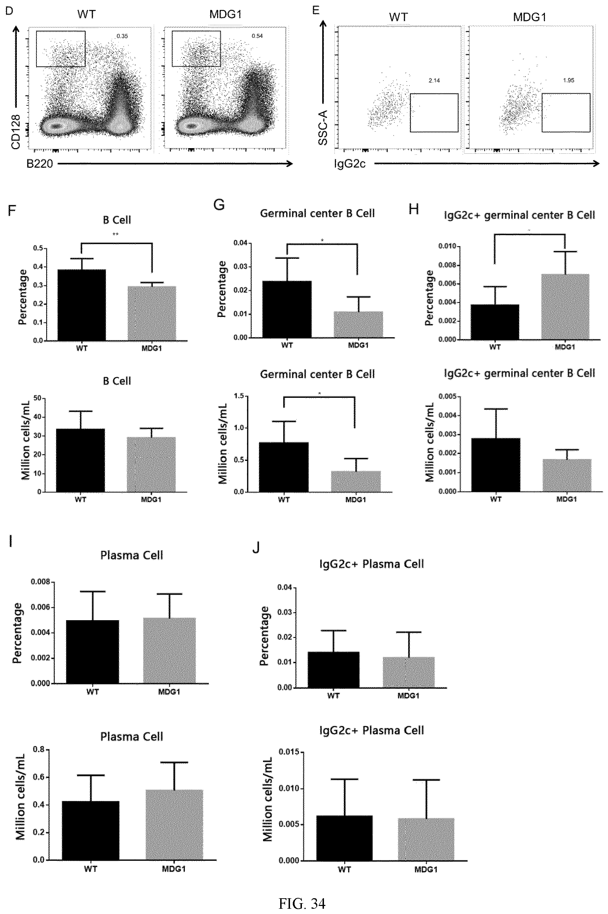

FIG. 34 shows growth of germinal center B cells and plasma cells in the spleens of a wild-type mouse and a MDG1 mouse after antigen immunization as mentioned in example 26 of the present application. FIGs. 34A-34E show growth states of spleen B cells, germinal center B cells, IgG2c + germinal center B cells, plasma cells, and IgG2c + plasma cells obtained after immunization, respectively; and FIGs. 34F-34J show the proportions and numbers of spleen B cells, germinal center B cells, IgG2c+ germinal center B cells, plasma cells, and IgG2c+ plasma cells obtained after immunization, respectively, wherein WT is a wild-type mouse, MDG1 is a homozygous knockout mouse, n = 6, statistical analysis is performed by using a two-tailed T test, * represents p < 0.05 and ** represents p < 0.01.

### Detailed Description of the Invention

In order to make the objects, technical solutions and advantages of the present application clearer, the technical solutions in the examples of the present application will be described clearly and completely below. Obviously, the embodiments described are some of, rather than all of, the embodiments of the present application. All the other examples obtained by a person of ordinary skill in the art on the basis of the examples of the present application without any creative effort shall fall within the scope of protection of the present application.

In addition, in order to better illustrate the present application, numerous specific details are given in the detailed description of embodiments below. It should be understood by a person skilled in the art that the present application is also implementable, without certain specific details. In some embodiments, materials, components, methods, means, etc. that are well known to a person skilled in the art are not described in detail, so as to highlight the gist of the present application.

Unless expressly stated otherwise, throughout the description and claims, the term "comprise" or variants thereof such as "contains" or "comprises" will be understood as including the stated elements or components, but not excluding other elements or components.

**The experimental materials used in the following examples and their sources are as follows:**
px330 plasmid vectors, purchased from Addgene, plasmid no. #58778;
pEASY-T5 Zero Cloning vectors, purchased from TransGen Biotech Co., Ltd., cat. no.: CT501-01;
TOP10 competent cells, purchased from Tiangen Biotech Co., Ltd., cat. no.: CB104;
PET28 vectors, purchased from Wuhan Miaoling Bioscience & technology Co., Ltd, cat. no.: P31003;
HiPure Total RNA Plus Mini Kit, derived from Magen Biotechnology Co., Ltd., cat. no.: R4121;
HiPure Gel Pure Micro Kit, derived from Magen Biotechnology, cat. no.: D2110;
HiPure Tissue DNA Mini Kit, derived from Magen Biotechnology, cat. no.: D3121;
2×M5 Hiper plus Taq HiFi PCR mix, derived from Mei5 Biotechnology Co., Ltd., cat. no.: MF002-plus;
Reverse transcription reagents 5X All-In-One RT MasterMix, derived from abmgood, cat. no.: 490;
DNA markers, derived from Guangzhou Dongsheng Biotech Co., Ltd., cat. no.: M1061/M1062);
Protein markers, derived from Thermo, cat. no.: 26617;
Hieff qPCR SYBR Green Master Mix from Yeasen, cat. no.: 11201ES08;
APC/Cy7 anti-mouse CD38 Antibody, derived from Biolegend, cat. no.: 102727;
APC/Cy7 anti-mouse IgM Antibody, derived from Biolegend, cat. no.: 406515;
CD45R (B220) Monoclonal Antibody (RA3-6B2), APC, derived from eBioscience, cat. no.: 85-17-0452-82;
Anti-CD23 antibody (Allophycocyanin), derived from Abeam, cat. no.: ab25457;
CD45R (B220) Monoclonal Antibody (RA3-6B2), eFluor 450, derived from eBioscience, cat. no.: 85-48-0452-82;
BV421 Rat Anti-Mouse CD138, derived from BD Horizon, cat. no.: 562610;
CD19 Monoclonal Antibody (eBio1D3 (1D3)), eFluor 506, derived from eBioscience, cat. no.: 85-69-0193-80;
CD43 Monoclonal Antibody (eBioR2/60), PE, derived from eBioscience, cat. no.: 85-12-0431-81;
CD23 Monoclonal Antibody (B3B4), PE, derived from eBioscience, cat. no.: 85-12-0232-82;
Anti-CD5 antibody [53-7.3] (Phycoerythrin), derived from Abeam, cat. no.: ab114078;
CD21/CD35 Monoclonal Antibody (eBio8D9 (8D9)), PE-Cyanine7, derived from eBioscience, cat. no.: 85-25-0211-80;
PE-Cy^{™}7 Hamster Anti-Mouse CD95, derived from BD Pharmingen, cat. no.: 553653;
CD19 Monoclonal Antibody PE-Cyanine7, derived from eBioscience, cat. no.: 25-0193-82;
IgM Monoclonal Antibody (eB121-15F9), PE-Cyanine7, derived from eBioscience, cat. no.: 85-25-5890-82;
CD43 Monoclonal Antibody (eBioR2/60), FITC, derived from eBioscience, cat. no.: 85-11-0431-85;
Mouse IgG2c Antibody - FITC Conjugated, derived from Aviva system biology, cat. no.: OASA06628;
7-AAD Viability Staining Solution, derived from eBioscience, cat. no.: 85-00-6993-50;
Goat Anti-Mouse IgG2c heavy chain (HRP), derived from Abeam, cat. no.: ab97255;
ECL Prime Western Blot Detection reagent, derived from GE, cat. no.: RPN2236;
Mouse IgM ELISA Quantitation Set, derived from bethyl, cat. no.: E90-101;
Mouse IgG2c ELISA Quantitation Set, derived from bethyl, cat. no.: E90-136;
Mouse IgG ELISA Quantitation Set, derived from bethyl, cat. no.: E90-131;
Mouse IgA ELISA Quantitation Set, derived from bethyl, cat. no.: E90-103;
Mouse IgE ELISA Quantitation Set derived from bethyl, cat. no.: E90-115;
TMB Substrate Set, derived from Biolegend, cat. no.: 421101;
Freund's Adjuvant complete, derived from sigma, cat. no.: F5881; and
Freund's Adjuvant incomplete, derived from sigma, cat. no.: F5506.

### Part I. Ighm-d-g homozygous mice and immunization results thereof

### Example 1. Preparation of Ighm-d-g homozygous mice (also referred to as MDG1 mice)

Ighm-d-g mice referred to C57BL/6 mice in which Ighm genes, Ighd genes, Ighg3 genes, Ighg1 genes, Ighg2b genes and first exons on Ighg2c gene were knocked out, wherein the first exons were responsible for encoding CH1 domains of IgG2c heavy chains. Ighm-d-g homozygous mice were produced by means of the following steps:
(1) Acquisition of nucleic acid molecules:
A schematic diagram of gene loci of heavy chain variable and constant regions of a C57BL/6 mouse antibody was as shown in FIG. 1, and Ighm, Ighd, Ighg3, Ighg1 and Ighg2b genes and a nucleotide sequence encoding a CH1 domain in Ighg2c in the mouse were knocked out by designing the targeting strategy as shown in FIG. 2. Targeting sequences (SEQ ID NO: 1 and SEQ ID NO: 2) for sgRNA were selected on the upstream portion of the first exon of the Ighm gene in the mouse, targeting sequences (SEQ ID NO: 3 and SEQ ID NO: 4) for sgRNA were selected on the downstream portion of the first exon of Ighg2c in the mouse, and sgRNA sequences were designed according to the targeting sequences. The details were as shown in Table 1.

**Table 1**

| **Tar get posi tion s for sgR NA** | **Targeting sequences for sgRNA (5' > 3')** | **Designed sgRNA sequences (5' > 3')** |
|---|---|---|
| Igh m | GACTCTCTGAG GACCAGAGA (SEQ ID NO: 1) | GACUCUCUGAGGACCAGAGAguuuaagagcuaugcuggaaacagcauag caaguuuaaauaaggcuaguccguuaucaacuugaaaaaguggcaccgagucggugcuu uuuuu (SEQ ID NO: 9) |
| | TGGGAAGGAC TGACTCTCTG (SEQ ID NO: 2) | GUGGGAAGGACUGACUCUCUGguuuaagagcuaugcuggaaacagcau agcaaguuuaaauaaggcuaguccguuaucaacuugaaaaaguggcaccgagucggugc uuuuuuu (SEQ ID NO: 10) |
| Igh g2c | AGTGGACAAG AAAATTGGTG( SEQ ID NO: 3) | GAGUGGACAAGAAAAUUGGUGguuuaagagcuaugcuggaaacagcau agcaaguuuaaauaaggcuaguccguuaucaacuugaaaaaguggcaccgagucggugc uuuuuuu (SEQ ID NO: 11) |
| | CTTGTGAACTT CTACTCCCC (SEQ ID NO: 4) | GCUUGUGAACUUCUACUCCCCguuuaagagcuaugcuggaaacagcaua gcaaguuuaaauaaggcuaguccguuaucaacuugaaaaaguggcaccgagucggugcu uuuuuu (SEQ ID NO: 12) |

(2) Construction of nucleic acid molecule into backbone plasmid and in-vitro transcription to obtain sgRNA:
The synthesized forward and reverse DNA oligos encoding an sgRNA sequence formed complementary double strands by means of annealing, the double strands were connected to an sgRNA expression vector (px330) by using T4 ligase, and after the connection, the resulting product was sequenced and verified by a professional sequencing company. The results showed that a target plasmid was obtained, and sgRNA was further obtained by in-vitro transcription.
(3) Introduction of sgRNA and Cas9 proteins into fertilized eggs of host animals:
The mice were subjected to ovulation induction and in-vitro fertilization, and the obtained fertilized eggs were cultivated. Then, the sgRNA and Cas9 proteins (or Cas9 mRNA, commercially available) were mixed and electroporated the fertilized eggs of the mice or injected together into the fertilized eggs of the mice by means of a microinjection method.
(4) Implantation of cells containing above-mentioned sgRNA and Cas9 proteins into host animals:
The above-mentioned fertilized egg cells were implanted into surrogate mother mice, and F0-generation chimeric mice can be produced. Individuals in which knockout occurred among the F0-generation mice were detected by extracting mouse tail genomic DNA and performing PCR detection. The knockout mice were sequenced so as to confirm that target sequences were deleted. F0-generation chimeric mice in which genes were correctly knocked out were selected for subsequent propagation and identification.

The genes which are knocked out can be detected by using PCR primers Ighm-d-g-1F and Ighm-d-g-1R (as indicated by arrows in FIG. 2), and wild-type genes can be detected by using primers Ighd-2F and Ighd-2R (as indicated by arrows in FIG. 2). The sequence of each primer was as shown in Table 2, and a schematic diagram of the PCR results of detecting individuals in which knockout occurred in Ighm-d-g mice was as shown in FIG. 3. The genes knocked out from Ighm-d-g homozygous mice were amplified by using primers Ighm-d-g-1F and Ighm-d-g-1R to obtain a target band with a size of about 700 bp (as shown in the figure on the left), and cannot amplified by using primers Ighd-2F and Ighd-2R to obtain a band (as shown in the figure on the right); Ighm-d-g wild-type genes cannot be amplified by using primers Ighm-d-g-1F and Ighm-d-g-1R to obtain a band (as shown in the figure on the left), and were amplified by using primers Ighd-2F and Ighd-2R to obtain a target band with a size of about 600 bp (as shown in the figure on the right).

**Table 2**

| **Primers** | **Sequences (5' > 3')** |
|---|---|
| Ighm-d-g-1F | GTTCATGCCCCTAGAGTTGG (SEQ ID NO: 17) |
| Ighm-d-g-1R | TACCATTCTCCCTGGAGTGG (SEQ ID NO: 18) |
| Ighd-2F | GCTCCTGAGACTGGCCATAG (SEQ ID NO: 19) |
| Ighd-2R | AAAAGGGAGGGAATGAATGG(SEQ ID NO: 20) |

(5) Propagation of heterozygous and homozygous knockout mice:
the F0-generation mice in which target genes were knocked out were mated with wild-type mice to obtain F1-generation mice, and by extracting genomes of mouse tails and performing PCR detection, F1-generation positive heterozygous knockout mice that can be stably inherited were selected. Then, the F1-generation heterozygous mice were mated with each other to obtain F2-generation positive homozygous knockout mice, i.e., Ighm-d-g homozygous mice. The method of identifying the genotypes of the obtained F1-generation heterozygous or F2-generation homozygous mice were the same as that in step (4).

### Example 2. Antigen immunization and titer determination

### Ighm-d-g homozygous mice were immunized with a human C-reactive protein (CRP).

An immunization method was as follows: 6- to 8-week-old male mice were selected, and the antigen human C-reactive protein (CRP, A-5172, Beijing Biobridge Biotechnology Co., Ltd) was emulsified with a freund's complete adjuvant (F5881, Sigma) with the equal volume to a state of no dripping and then was used for subcutaneous multi-point injection for first immunization of mice, wherein the injected dose was 100 µg/mouse in the first immunization. After the first immunization, subsequent subcutaneous immunization was performed every 2 weeks, wherein the CRP antigen was emulsified with a Freund's incomplete adjuvant (F5506, Sigma) and then was used for subcutaneous multi-point injection of mice, and the injected dose was 100 µg/mouse every time.

A method for determining a serum titer was as follows: a CRP antigen was diluted to 2 µg/mL, 100 µL of diluted antigen was taken and added to a polystyrene enzyme-linked assay plate, the plate was covered, and a specific IgG2c antibody heavy chain that specifically binds to the CRP antigen in serum was detected by using HRP-goat anti-mouse IgG-Fc (Jackson 115-035-071).

As shown in FIG. 4, blood was collected when the mice was unimmunized, one week after the first immunization, one week after the second immunization, and one week after the third immunization. The serum was subjected to doubling dilution with PBS, starting from a dilution of 1: 500. The serum titer was detected by ELISA, and the results showed that no specific antibody binding to the antigen CRP exist in the mice when the mice was unimmunized and after the first immunization. After the second immunization, an IgG2c antibody that specifically binds to the antigen exists in the mice, and the titer was not further increased after the third immunization, wherein the serum titer was around 1: 8000, which can be used for an antibody gene extraction experiment in the next step.

### Example 3. Examination of spleens, thymus glands, lymph nodes and organs of unimmunized Ighm-d-g homozygous mice and CRP antigen-immunized Ighm-d-g homozygous mice

Following example 2 above, the mice were euthanized by causing asphyxiation using carbon dioxide, and dissected to observe the thymus glands, spleens, mesenteric lymph nodes, submaxillary lymph nodes, etc. and the sizes and shapes of the heart, liver, lung, kidney and other organs. The dissection results showed that the thymus glands, mesenteric lymph nodes, submaxillary lymph nodes and major organs of unimmunized Ighm-d-g homozygous mice and antigen-immunized Ighm-d-g homozygous mice all had no obvious abnormalities.

### Example 4. Specific IgG2c heavy chain antibody produced by means of antigen immunization

Following example 2 above, Western blot was performed on the serum of the CRP antigen-immunized mice: 2 µL of serum was taken from the immunized mice, 100 µL of PBS was added, the resulting mixture was reacted with 10 µL of CRP antigen-Sepharose packings at room temperature for 60 min, then the resulting product was centrifuged at 6000 rpm for 30 s, and the supernatant was discarded; and the packings were washed three times with PBS, resuspended with 10 µL of PBS, boiled, electrophoresed by using 12% SDS-PAGE, transferred to a PVDF membrane, and then reacted with an HRP-goat anti-mouse IgG-Fc (Jackson 115-035-071, for detection of a heavy chain) antibody and an HRP-goat anti-mouse Ig light chain (Jackson, 115-035-174) antibody respectively, and further color development was performed.

According to the design of the present application, since the IgM, IgD, IgG1, IgG2b and IgG3 genes in the mice were knocked out, only an IgG2c isotype antibody can be produced by using antigen protein-immunized Ighm-d-g homozygous mice, and since the CH1 gene of the IgG2c heavy chain was knocked out, an IgG2c heavy chain antibody (free of a CH1 domain) can be produced by using the antigen protein-immunized Ighm-d-g homozygous mice, and the molecular weight of one IgG2c heavy chain alone was about 40 KD. The antibody produced by the antigen protein-immunized Ighm-d-g homozygous mice was subjected to separation, electrophoresis, color development and other steps, then a band that specifically binds to the antigen CRP existed around a band of about 80 KD, which is consistent with the theoretical dimer molecular weights of the IgG2c heavy chain, and a dimer not bound to an HRP-goat anti-mouse Ig light chain (Jackson, 115-035-174) antibody was included.

### Example 5. Extraction of IgG2c heavy chain antibody genes

Following example 2 above, the Ighm-d-g homozygous mice were immunized with a CRP antigen and the serum titer was determined. Then, the mice were euthanized, splenocytes were taken and lysed with Trizol, total RNA was extracted and reversely transcribed to obtain cDNA, and a heavy chain variable region and a heavy chain constant region connected thereto were amplified by PCR by using the following IgG2c isotype antibody-specific primers.
MHV1: ATGAAATGCAGCTGGGGCATSTTCTTC (SEQ ID NO: 21);
MHV2: ATGGGATGGAGCTRTATCATSYTCTT (SEQ ID NO: 22);
MHV3: ATGAAGWTGTGGTTAAACTGGGTTTTT (SEQ ID NO: 23);
MHV4: ATGRACTTTGGGYTCAGCTTGRTTT (SEQ ID NO: 24);
MHV5: ATGGGACTCCAGGCTTCAATTTAGTTTTCCTT (SEQ ID NO: 25);
MHV6: ATGGCTTGTCYTTRGSGCTRCTCTTCTGC (SEQ ID NO: 26);
MHV7: ATGGRATGGAGCKGGRGTCTTTMTCTT (SEQ ID NO: 27);
MHV8: ATGAGAGTGCTGATTCTTTTGTG (SEQ ID NO: 28);
MHV9: ATGGMTTGGGTGTGGAMCTTGCTTATTCCTG (SEQ ID NO: 29);
MHV10: ATGGGCAGACTTACCATTCTCATTCCTG (SEQ ID NO: 30);
MHV11: ATGGATTTTGGGCTGATTTTTTTTATTG (SEQ ID NO: 31);
MHV12: ATGATGGTGTTAAGTCCTTCTGTACC (SEQ ID NO: 32); and B6_IgG2c_CH2 R1:5'- TGGTCCACCCAAGAGGTCTG-3' (SEQ ID NO: 33);

The PCR reaction system was as shown in Table 3:

**Table 3**

| | |
|---|---|
| Template cDNA | 5 µL |
| Mixture of MHV1-12 with equivalent amounts | 2 µL |
| B6-IgG2c-CH2R1 | 2 µL |
| I-5^{™} 2×High-Fidelity Master Mix (Tsingke Biotechnology Co., Ltd. cat. no.: TP001) | 25 µL |
| ddH₂O | 16 µL |

PCR reaction process: 98°C, 2 min - 30 cycles (98°C each cycle, 10 s - 50°C, 20 s - 72°C, 40 s) - 72°C, 5 min - 16°C.

The obtained PCR amplification product was connected with a pEASY-T5 Zero Cloning vector, transformed TOP10 competent cells and coated an LB plate. 11 clones were randomly selected, and the clones identified as positive by means of colony PCR were sent for sequencing (as shown in FIG. 5A, the band of 650 bp was a positive band).

The sequencing results of the positive clones showed (as shown in FIG. 5B) that FR4 regions of the IgG2c antibody heavy chain variable regions produced in Ighm-d-g homozygous mice were directly connected to IgG2c Hinge regions (antibody hinge regions), and the structures of the antibody heavy chains were VH-CH1-Hinge-CH2-CH3, wherein the VH region can be further divided into FR1-CDR1-FR2-CDR2-FR3-CDR3-FR4 in detail. As can be seen from FIG. 5B, FR4 regions of the IgG2c antibody heavy chain variable regions produced in the Ighm-d-g homozygous mice were directly connected to the Hinge regions (the antibody hinge regions), indicating that the IgG2c CH1 exons were successfully knocked out.

### Example 6. Phage display library construction and panning of CRP antigen-specific IgG2c heavy chain antibody variable regions

### I. Phage display library construction

Following example 2 above, total RNA was extracted from the spleens of CRP-immunized Ighm-d-g homozygous mice, cDNA (a TAKAR 6110A cDNA synthesis kit) was prepared by using oligo (Dt), and heavy chain antibody genes were obtained by two rounds of PCR amplification using nested PCR.

The first round of PCR process was exactly the same as the PCR process mentioned in example 5; and
the primers, reaction system and reaction process used in the second round of PCR were as follows:

MHVF1-SfiI: ATGCCATGACTGTggcccaggcggcc GAG GTG AAG CTT CTC GAG TCT
GG (SEQ ID NO: 34);

MHVF2-SfiI: CATGCCATGACTGTggcccaggcggcc SAG GTS CAG CTG MAG GAG TCW
GG (SEQ ID NO: 35);

MHVF3-SfiI: CATGCCATGACTGTggcccaggcggcc GAG GTC CAG CTG CAA CAA TCT
GG (SEQ ID NO: 36);

MHVF4-SfiI: CATGCCATGACTGTggcccaggcggcc SAG GTY CAR CTK CAG CAG YCT
GG (SEQ ID NO: 37);

MHVF5-SfiI: CATGCCATGACTGT ggcccaggcggcc GAR GTG AAG CTT GWG GAG
TCT GG (SEQ ID NO: 38);

B6-IgG2c-Hin-sfi1: ACTCGCGGCCGGCCTGGCCTGTTATGGGCACTCTGGG (SEQ ID NO: 39).

The reaction system was as shown in Table 4:

**Table 4**

| | |
|---|---|
| The product recovered in the first round of PCR as a template. | 2 µL |
| Mixture of MHVF1-5-SfiI with equivalent amounts | 2 µL |
| B6-IgG2c-Hin-sfi1 | 2 µL |
| I-5^{™} 2×High-Fidelity Master Mix (Tsingke Biotechnology Co., Ltd. cat. no.: TP001) | 25 µL |
| ddH₂O | 16 µL |

PCR reaction process: 98°C, 2 min - 30 cycles (98°C each cycle, 10 s - 65°C, 20 s - 72°C, 40 s) - 72°C, 5 min - 16°C.

The PCR amplification product was digested with SfiI (FD1824, Thermo) and subjected to agarose gel electrophoresis, the target product was subjected to gel extraction, and then the target fragment subjected to gel extraction was cloned into a pComb3XSS vector digested with SfiI. R2738 electrocompetent cells were electroporated. An IgG2c heavy chain antibody library was prepared and multiple rounds of panning were performed. Multiple positive clones were selected and sent for sequencing identification, and the results were as shown in FIG. 6.

### II. Panning

### 1. panning

1) Coating: A CRP antigen was diluted to 100 µg/mL with a coating solution and added to 2 wells of an enzyme-linked assay plate (100 µL/well), and overnight coating was performed at 4°C.
2) Blocking: The coating solution was aspirated, the plate was washed 3 times with PBS and blocked with 300 µL of 4% skim milk (the blocking solution for the second, third, fourth, and fifth rounds was 4% BSA), and incubation was performed at 37°C for 2 h.
3) Binding: The blocking solution was aspirated, the plate was washed 3 times with PBS, 100 µL of phage display library was added, and incubation was performed at 37°C for 1 h.
4) Washing: unbound phages were aspirated, and PBST was added for washing (five rounds of panning were performed in the present application, PBST was added for washing 3 times in the first and second rounds respectively, and PBST was added for washing 10 times in the third, fourth and fifth rounds respectively).
5) Elution: 100 µL of Gly-HCl (pH 3.0) was added, 37°C, 5 min and the resulting mixture was gently pipetted with a pipettor and beat several times.
6) Neutralization: the liquid in the wells was aspirated to a centrifuge tube, 15 µL of neutralization buffer (1M Tirs-Hcl, PH = 8.8) was added in advance, and the resulting mixture was mixed uniformly.
7) 10 µL of eluent obtained after neutralization was taken, the titer was determined, and the remaining eluent was amplified, cultured and then used in the next round of panning.

### 2. Phage amplification and purification

1) The eluent obtained after neutralization was added to 5 mL of ER2738 bacterial solution (commercially purchased strain, wherein OD₆₀₀ was about 0.5-0.7), and the resulting mixture was mixed uniformly;
2) the mixed mixture was left to stand in a 37°C incubator for 30 min, and was then cultured at 180 rpm at 37°C for 1 h;
3) the obtained culture was added to a 20 mL of LB culture medium and cultured at 180 rpm at 37°C for 2h;
4) 20 µL of helper phage M13KO7 (2 × 10⁹ cfu) (purchased from NEB Company, cat. No.: N03158) was added, and the resulting mixture was mixed uniformly;
5) the mixed mixture was left to still stand in a 37°C incubator for 30 min and was cultured at 180 rpm at 37°C for 1 h, and then ampicillin antibiotics were added;
6) the mixture was cultured at 180 rpm at 37 °C for 1 h, and then the mixture was centrifuged at 8000 rpm for 5 min;
7) the precipitate was resuspended with 25 mL of LB culture medium/Amp/kan and cultured at 180 rpm at 30°C overnight (about 14 h);
8) the resulting product was centrifuged at 8000 rpm at 4°C for 10 min and the supernatant was taken;
9) a PEG-NaCl solution with 1/5 volume was added, and the resulting mixture was left to stand at 4°C for about 4-6 h;
10) after that, the mixture was centrifuged at 12000 rpm at 4°C for 20 min, and the supernatant was discarded; and
11) 1 mL of precipitate was resuspended, and 10 µL of resuspended precipitate was taken for titer determination.

### 3. Titer determination

1) The phages to be tested were diluted with PBS (10 µL of phages were added to 990 µL of PBS, which was 10⁻²), 10 µL of the diluted phages were taken and added to 200 µL of ER2738 bacterial solution respectively, and the resulting mixture was mixed uniformly;
2) the infected culture was statically cultured at 37°C for 30 min;
3) the resulting product coated a LB+Amp⁺ resistant plate;
4) inverted overnight incubation was performed at 37°C; and
5) the colonies were counted and the titer was calculated.

III. An IgG2c heavy chain antibody variable region library was prepared and subjected to five rounds of panning, and the results were as shown in Table 5 below:

**Table 5. CRP-MDG1 screening data sheet**

| Rounds | Coating amount of antigen | Amount of phages added | Times of number of PBST washing | Phage output | Enrichment degree |
|---|---|---|---|---|---|
| 1 | 10 µg | 1.5 × 10¹⁰ cfu | 3 | 6 × 10³ cfu | |
| 2 | 10 µg | 1.5 × 10¹⁰ cfu | 3 | 6 × 10⁶ cfu | 1000 |
| 3 | 10 µg | 1.5 × 10¹² cfu | 10 | 1 × 10⁸ cfu | 16.7 |
| 4 | 10 µg | 1 × 10¹² cfu | 10 | 1 × 10⁹ cfu | 10 |
| 5 | 10 µg | 3 × 10¹² cfu | 10 | 2 × 10⁹ cfu | 2 |

A total of 5 rounds of panning were performed; as can be seen from the table above, the output phages were significantly enriched; and positive clones were selected by means of PHAGE-ELISA experiments and sent for sequencing identification.

### Example 7. Prokaryotic expression and biological activity identification of CRP antigen-specific IgG2c heavy chain antibody variable region

Following example 6 above, the sequences for sequencing were analyzed, and D4-12 (the nucleotide sequence and amino acid sequence were as shown in Table 6) that appeared repeatedly in the first five rounds of panning was selected to transfect BL21 Escherichia coli, Different concentrations of IPTG were added at 16°C, prokaryotic expression was induced, ultrasonication was performed, and the supernatant was collected and purified by means of a nickel affinity column.

A specific antigen CRP and an unrelated protein OVA (2 µg/mL) were coated, gradient dilution was performed by using purified D4-12, and ELISA detection was performed. The results showed that a variable region of the purified D4-12 heavy chain antibody can specifically recognize and bind to the antigen CRP and showed a good concentration dependence, as shown in FIG. 7.

**Table 6**

| Antibody no. | Nucleotide sequence | Amino acid sequence |
|---|---|---|
| D4-12 | GAGGTCCAGCTGCAACAATCTGGACCTGAGTT GGTGAAGCCTGGGGCTTCAGTGAAGATTTCCT GTAAGGCTTCTGGATACATGTTCAGTGACTACT ATATGAATTGGGTGAAGCAGAGCCATGGAAAG AGCCTTGAGTGGATTGGAAATGTTGATCCTAA CACTGATAATACTATATACAACCAGAACTTCAA GGACAAGGCCACATTGACTGTAGACAGGTCCT CCAGAACAGCCTACATGGAACTCCACAGCCTG ACATCTGAGGACTCTGCAGTCTATTACTGTGCA AGATCGAGGTTCTATGATCCCTGGTTTGCTTAC TGGGGCCAAGGGACTCTGGTCACTGTCTCTGC A (SEQ ID NO: 40) | EVQLQQSGPELVKPGASVKIS CKASGYMFSDYYMNWVKQ SHGKSLEWIGNVDPNTDNTI YNQNFKDKATLTVDRSSRTA YMELHSLTSEDSAVYYCARS RFYDPWFAYWGQGTLVTVSA (SEQ ID NO: 41) |

### Example 8. Eukaryotic expression and biological activity identification of CRP antigen-specific IgG2c heavy chain antibody variable region

Following example 6 above, the sequences for sequencing were further analyzed, and the sequence D5S-12 (the nucleotide sequence and amino acid sequence were as shown in Table 7) with the most repetitions was selected for eukaryotic expression and biological activity identification. KOP293 cells (Zhuhai Kairui) were transfected, the supernatant of cultured cells was collected on day 6, and a target protein was purified by means of a nickel affinity column.

**Table 7**

| Antibody no. | Nucleotide sequence | Amino acid sequence |
|---|---|---|
| D5S-12 | GAGGTGAAGCTTCTCGAGTCTGGGGGAGGCTT AGTGAAGCCTGGAGGGTCCCTGAAACTCTCCT GTGCAGCCTCTGGATTCACTTTCAGTGACTATG GCATGTCTTGGATTCGCCAGACTCCAGACAAG AGGCTGGAGTGGGTCGCAACCATTAGTAGTGG TAATACTTACACCTATTATTCAAAAAGTGTGAA GGGGCGATTCACCATCTCCAGAGACAATGCCA AGAACACCCTGTACCTGCAAATGAGCAGTCTG AAGTCTGAAGACACAGCCATATATTACTGTTCA AGACATACCTCTGATTATTACCCTCTGAGCCAA GGGACTCTGGTCACTGTCTCTGCA (SEQ ID NO: 42) | EVKLLESGGGLVKPGGSLKL SCAASGFTFSDYGMSWIRQT PDKRLEWVATISSGNTYTYYS KSVKGRFTISRDNAKNTLYL QMSSLKSEDTAIYYCSRHTSD YYPLSQGTLVTVSA (SEQ ID NO: 43) |

1. A specific antigen CRP and an unrelated protein OVA or BSA (2 µg/mL) were coated, and the purified D5S-12 antibody was subjected to gradient dilution and detected by means of ELISA. The results showed that the purified D5S-12 heavy chain antibody can specifically recognize and bind to the antigen CRP and showed a good concentration dependence, as shown in FIG. 8.
2. Determination of affinity of D5S-12 heavy chain antibody: The affinity of the purified D5S-12 heavy chain antibody to the antigen CRP was determined by using FORTEBIO-OCTET, the antibody was diluted to 10 µg/mL and the antigen CRP was diluted to 200 nM, 100 nM, 50 nM, 25 nM, 12.5 nM, 6.25 nM and 3.13 nM. The affinity constants of the antibody were determined as K_{D}(M) = 9.87E-10, Kₒₙ(1/Ms) = 8.55E+04 and K_{off}(1/s) = 8.44E-05, and the heavy chain antibody D5S-12 had high affinity to the antigen CRP. The results were as shown in FIG. 9.

### Part II. Preparation of IghM-3G3 homozygous mice and immunization results thereof

IghM-3G3 mice were C57BL/6 mice in which the first exon on the Ighm gene was knocked out, and the Ighg3 gene, the Ighg1 gene, the Ighg2b gene and the first exon located on the Ighg2c gene were knocked out. IghM homozygous mice (i.e., C57BL/6 mice where the first exon on the Ighm gene was knocked out) were produced first, and then IghM-3G3 homozygous mice were produced on the basis of the IghM homozygous mice.

### Example 9. Preparation of IghM homozygous mice

Preparation of IghM homozygous mice comprises the following steps:
(1) Acquisition of nucleic acid molecules:
   A schematic diagram of the genetic loci of the heavy chain variable and constant regions of a C57BL/6 mouse antibody was as shown in FIG. 1, and a nucleotide sequence encoding a CH1 domain in a mouse Ighm gene was knocked out by designing the targeting strategy as shown in FIG. 10.

In order to knock out the nucleotide sequence encoding the CH1 domain in the mouse Ighm gene, targets were selected on the upstream and downstream portions of the first exon in the mouse Ighm gene. Targeting sequences (SEQ ID NO: 1 and SEQ ID NO: 2) for sgRNA were selected on the upstream portion of the first exon of the Ighm gene in the mouse, targeting sequences (SEQ ID NO: 5 and SEQ ID NO: 6) for sgRNA were selected on the downstream portion of the first exon of Ighm in the mouse, and sgRNA sequences were designed according to the targeting sequences. The details were as shown in Table 8.

**Table 8**

| **Target position s for sgRNA** | **Targeting sequences for sgRNA (5' > 3')** | **sgRNA sequences designed** |
|---|---|---|
| Ighm | GACTCTCTGAGGAC CAGAGA (SEQ ID NO: 1) | GACUCUCUGAGGACCAGAGAguuuaagagcuaugcuggaaacagcaua gcaaguuuaaauaaggcuaguccguuaucaacuugaaaaaguggcaccgagucggugc uuuuuuu (SEQ ID NO: 9) |
| | TGGGAAGGACTGA CTCTCTG (SEQ ID NO: 2) | GUGGGAAGGACUGACUCUCUGguuuaagagcuaugcuggaaacagcau agcaaguuuaaauaaggcuaguccguuaucaacuugaaaaaguggcaccgagucggugc uuuuuuu (SEQ ID NO: 10) |
| Ighm | GATCTGCATGTGCC CATTCC (SEQ ID NO: 5) | GAUCUGCAUGUGCCCAUUCCguuuaagagcuaugcuggaaacagcauag caaguuuaaauaaggcuaguccguuaucaacuugaaaaaguggcaccgagucggugcu uuuuuu (SEQ ID NO: 13) |
| | GGTTTGGTTCTTAC CTGGAA (SEQ ID NO: 6) | GGUUUGGUUCUUACCUGGAAguuuaagagcuaugcuggaaacagcaua gcaaguuuaaauaaggcuaguccguuaucaacuugaaaaaguggcaccgagucggugc uuuuuuu (SEQ ID NO: 14) |

(2) Construction of nucleic acid molecule into backbone plasmid and in-vitro transcription to obtain RNA:
   The synthesized forward and reverse DNA oligos for an sgRNA sequence formed complementary double strands by means of annealing, the double strands were connected to an sgRNA expression vector (px330) by using T4 ligase, and after the connection, the resulting product was sequenced and verified by a professional sequencing company. The results showed that a target plasmid was obtained, and sgRNA was further obtained by in-vitro transcription.
(3) Introduction of sgRNA and Cas9 proteins into fertilized eggs of host animals:
   The mice were subjected to ovulation induction and in-vitro fertilization, and the obtained fertilized eggs were cultivated. Then, the sgRNA and Cas9 proteins (or Cas9 mRNA, commercially available) were mixed and electroporated the fertilized eggs of the mice or injected together into the fertilized eggs of the mice by means of a microinjection method.
(4) Implantation of cells containing above-mentioned sgRNA and Cas9 proteins into surrogate animals:
   The above-mentioned fertilized egg cells were implanted into surrogate mother mice, and F0-generation chimeric mice can be produced. Individuals in which knockout occurred among the F0-generation mice were detected by extracting mouse tail genomic DNA and performing PCR detection. The knockout mice were sequenced so as to confirm that target sequences were deleted. F0-generation chimeric mice in which genes were correctly knocked out were selected for subsequent propagation and identification.

The genes which are knocked out (as indicated by arrows in FIG. 10) can be detected by using PCR primers Ighm-F and Ighm-R, and see table 9 for the primer sequences.

**Table 9**

| Primers | Sequences (5' > 3') |
|---|---|
| Ighm-F | CTGTGGCTAGAAGGCAGCTC (SEQ ID NO: 44) |
| Ighm-R | ATCTCTGCGACAGCTGGAAT (SEQ ID NO: 45) |

The test results were as shown in FIG. 11. When PCR detection was performed by using the primers above, the Ighm wild-type genes can be amplified to obtain a band of about 600 bp, and the Ighm CH1 genes which are knocked out can be amplified to obtain a band of about 300 bp. As can be seen from FIG. 11, clones #1, #2, #3 and #6 contain Ighm CH1 genes which are knocked out. The knockout mice were sequenced so as to confirm that the first exons of the Ighm genes of the knockout mice were deleted.

(5) Propagation of heterozygous and homozygous knockout mice:
the F0-generation mice in which target genes were knocked out were mated with wild-type mice to obtain F1-generation mice, and by extracting genomes of mouse tails and performing PCR detection, F1-generation positive heterozygous knockout mice that can be stably inherited were selected. Then, the F1-generation heterozygous mice were mated with each other to obtain F2-generation positive homozygous knockout mice, i.e., IghM homozygous mice. The method of identifying the genotypes of the obtained F1-generation heterozygous or F2-generation homozygous mice were the same as that in step 4.

### Example 10. Extraction of IgM heavy chain antibody genes

Following example 2 above, IghM homozygous mice were immunized with a CRP antigen and then euthanized, splenocytes were taken and lysed with Trizol, total RNA was extracted and reversely transcribed to obtain cDNA, and a heavy chain variable region and a heavy chain constant region connected thereto were amplified by PCR by using the IgM isotype antibody-specific primers. The primers used and sequences thereof were as follows:
See example 5 for MHV1, MHV2, MHV3, MHV4, MHV5, MHV6, MHV7, MHV8, MHV9, MHV10, MHV11 and MHV12; and
B6 IghM CH2 R4: GTTCATCTCTGCGACAGC(SEQ ID NO:46).

The PCR reaction system was as shown in Table 10:

**Table 10**

| | |
|---|---|
| Template cDNA | 5 µL |
| Mixture of MHV1-12 with equivalent amounts | 2 µL |
| B6- IghM CH2 R4 | 2 µL |
| I-5^{™} 2×High-Fidelity Master Mix (Tsingke Biotechnology Co., Ltd. cat. no.: TP001) | 25 µL |
| ddH₂O | 16 µL |

PCR reaction process: 98°C, 2 min - 30 cycles (98°C each cycle, 10 s - 50°C, 20 s - 72°C, 40 s) - 72°C, 5 min - 16°C.

The PCR amplification product was connected to a pEASY-T5 Zero Cloning vector and transfected TOP10 competent cells, and the transfected cells were used to coat an LB plate (amphicillin-resistant), and the clones were picked for colony PCR. The positive clones were sent for sequencing. As can be ween from the sequencing results, FR4 in the heavy chain variable region of the IgM antibody expressed in the IghM homozygous mice was directly connected to CH2 (starting amino acid sequence AVAEMN). The results as were shown in FIG. 12, the exon of IgM CH1 in the genome was successfully knocked out.

### Example 11. Preparation of IghM-3G3 homozygous mice

A method for preparing IghM-3G3 homozygous mice, comprises the following steps:
(1) Acquisition of nucleic acid molecules:
   A schematic diagram of the gene loci of the heavy chain variable region and heavy chain constant region of a C57BL/6 mouse antibody was as shown in FIG. 1, and the Ighg3 gene, Ighg1 gene, Ighg2b gene and the first exon located on the Ighg2c gene were further knocked out by designing the targeting strategy as shown in FIG. 13 on the basis of the IghM homozygous mice obtained in example 9.

In order to knock out the first exon encoding a CH1 domain on the mouse Ighm, the Ighg3, Ighg1 and Ighg2b genes and the first exon encoding a CH1 domain on Ighg2c, on the basis of the IghM homozygous mice, targeting sequences (SEQ ID NO: 7 and SEQ ID NO: 8) for sgRNA were selected on the upstream portion of the first exon of the Ighg3 gene in the mouse, targeting sequences (SEQ ID NO: 3 and SEQ ID NO: 4) for sgRNA were selected on the downstream portion of the first exon of Ighg2c in the mouse, and sgRNA sequences were designed according to the targeting sequences. The details were as shown in Table 11.

**Table 11**

| **Tar get posi tion s for sgR NA** | **Targeting sequences for sgRNA (5' > 3')** | **sgRNA sequences designed** |
|---|---|---|
| Ighg 3 | TGTTGTAGCTGCAA GATAGG (SEQ ID NO: 7) | GUGUUGUAGCUGCAAGAUAGGguuuaagagcuaugcuggaaaca gcauagcaaguuuaaauaaggcuaguccguuaucaacuugaaaaaguggcaccga gucggugcuuuuuuu (SEQ ID NO: 15) |
| | GAGTGAGGTTATCA GACAGC (SEQ ID NO: 8) | GAGUGAGGUUAUCAGACAGCguuuaagagcuaugcuggaaacag cauagcaaguuuaaauaaggcuaguccguuaucaacuugaaaaaguggcaccgag ucggugcuuuuuuu (SEQ ID NO: 16) |
| Ighg 2c | AGTGGACAAGAAA ATTGGTG (SEQ ID NO: 3) | GAGUGGACAAGAAAAUUGGUGguuuaagagcuaugcuggaaaca gcauagcaaguuuaaauaaggcuaguccguuaucaacuugaaaaaguggcaccga gucggugcuuuuuuu (SEQ ID NO: 11) |
| | CTTGTGAACTTCTA CTCCCC (SEQ ID NO: 4) | GCUUGUGAACUUCUACUCCCCguuuaagagcuaugcuggaaaca gcauagcaaguuuaaauaaggcuaguccguuaucaacuugaaaaaguggcaccga gucggugcuuuuuuu (SEQ ID NO: 12) |

(2) Construction of nucleic acid molecule into backbone plasmid and in-vitro transcription to obtain RNA:
   The synthesized forward and reverse DNA oligos for an sgRNA sequence formed complementary double strands by means of annealing, the double strands were connected to an sgRNA expression vector (px330) by using T4 ligase, and after the connection, the resulting product was sequenced and verified by a professional sequencing company. The results showed that a target plasmid was obtained, and sgRNA was further obtained by in-vitro transcription.
(3) Introduction of sgRNA and Cas9 proteins into fertilized eggs of host animals:
   The mice were subjected to ovulation induction and in-vitro fertilization, and the obtained fertilized eggs were cultivated. Then, the sgRNA and Cas9 proteins (or Cas9 mRNA, commercially available) were mixed and electroporated the fertilized eggs of the mice or injected together into the fertilized eggs of the mice by means of a microinjection method.
(4) Implantation of cells containing above-mentioned sgRNA and Cas9 proteins into host animals:
   The above-mentioned fertilized egg cells were implanted into surrogate mother mice, and F0-generation chimeric mice can be produced. Individuals in which knockout occurred among the F0-generation mice were detected by extracting mouse tail genomic DNA and performing PCR detection. The knockout mice were sequenced so as to confirm that target sequences were deleted. F0-generation chimeric mice in which genes were correctly knocked out were selected for subsequent propagation and identification.

The genes which are knocked out can be detected by using PCR primers Ighg-1F and Ighg-1R, and the wild-type genes can be detected by using primers Ighg-2R and Ighg-1F (arrows in FIG. 13). The sequences of the primers were as shown in Table 12.

**Table 12**

| **Primers** | **Sequences (5' > 3')** |
|---|---|
| Ighg-1F | GGAATCAAAGGACAGCAGGA (SEQ ID NO: 74) |
| Ighg-1R | AGTGTCACCGAGGATCCAGA (SEQ ID NO: 75) |
| Ighg-2R | CCAAACAGGGCTTGGAGACT (SEQ ID NO: 76) |

IghM-3G3 mice were subjected to genotype identification by means of PCR (FIG. 14), and the Ighm genotype was identified by using primers Ighm-F and Ighm-R: the Ighm wild-type genes can be amplified to obtain a target product of about 600 bp and the genes which are knocked out can be amplified to obtain a target product of about 300 bp. The Ighg genotype was identified by using primers Ighg-1F, Ighg-1R and Ighg-2R: the Ighg genes which are knocked out can be amplified by using primers Ighg-1F and Ighg-2R to obtain a target product of about 500 bp, but produce no bands after being amplified by using primers Ighg-1F and Ighg-1R; and the Ighg wild-type genes produce no bands after being amplified by using primers Ighg-1F and Ighg-2R, but can be amplified by using primers Ighg-1F and Ighg-1R to obtain a target product of about 460 bp, wherein +/-: heterozygote; wt: wild type; -/-: knockout homozygote.

(5) Propagation of heterozygous and homozygous knockout mice: the F0-generation mice in which target genes were knocked out were mated with wild-type mice to obtain F1-generation mice, and by extracting genomes of mouse tails and performing PCR detection, F1-generation positive heterozygous knockout mice that can be stably inherited were selected. Then, the F1-generation heterozygous mice were mated with each other to obtain F2-generation positive homozygous knockout mice, i.e., IghM-3G3 homozygous mice. The method of identifying the genotypes of the obtained F1-generation heterozygous or F2-generation homozygous mice were the same as that in step 4.

### Example 12. Antigen immunization and titer determination for IghM-3G3 homozygous mice

IghM-3G3 homozygous mice were immunized with a human C-reactive protein (CRP).

An immunization method was as follows: 6- to 8-week-old male mice were selected, and the antigen human C-reactive protein (CRP, A-5172, Beijing Biobridge Biotechnology Co., Ltd) was emulsified with a freund's complete adjuvant (F5881, Sigma) with made a state of no dripping and then was used for subcutaneous multi-point injection for first immunization of mice, wherein the injected dose was 100 µg/mouse in the first immunization. After the first immunization, subsequent subcutaneous immunization was performed every 2 weeks, wherein the CRP antigen was emulsified with a Freund's incomplete adjuvant (F5506, Sigma) and then was used for subcutaneous multi-point injection of mice, and the injected dose was 100 µg/mouse every time.

### Serum titer determination:

A method for determining a serum titer was as follows: a CRP antigen was diluted to 2 µg/mL, 100 µL of diluted antigen was taken and added to a polystyrene enzyme-linked assay plate, the plate was covered, and a specific IgG antibody that specifically binds to the CRP antigen in serum was detected by using HRP-goat anti-mouse IgG-Fc (Jackson, 115-035-071).

As shown in FIG. 15, blood was collected when the mice was unimmunized, one week after the first immunization and one week after the third immunization. The serum was subjected to doubling dilution with PBS, starting from a dilution of 1: 500. The serum titer was detected by ELISA, and the results showed that no specific antibody binding to the antigen CRP exist in the mice when the mice was unimmunized and after the first immunization, and after the third immunization, the serum titer thereof was about 1: 32000.

### Example 13. Extraction of IgG2c heavy chain antibody genes

The IghM-3G3 homozygous mice were immunized with a CRP antigen and the serum titer was determined. Then, the mice were euthanized, splenocytes were taken and lysed with Trizol, total RNA was extracted and reversely transcribed to obtain cDNA, and a heavy chain variable region and a heavy chain constant region connected thereto were amplified by PCR by using the following IgG2c isotype antibody-specific primers.

See example 5 for MHV1, MHV2, MHV3, MHV4, MHV5, MHV6, MHV7, MHV8, MHV9, MHV10, MHV11, MHV12 and B6_IgG2c_CH2 R1.

The PCR reaction system and the PCR reaction process were as shown in example 5.

The obtained PCR amplification product was connected with a pEASY-T5 Zero Cloning vector, transformed TOP10 competent cells and coated an LB plate. 7 clones were randomly selected, and the clones identified as positive by means of colony PCR were sent for sequencing (as shown in FIG. 16A, the band of 650 bp was a positive band).

The sequencing results of the positive clones showed (as shown in FIG. 16B) that FR4 regions of the IgG2c antibody heavy chain variable regions produced in IghM-3G3 homozygous mice were directly connected to IgG2c Hinge regions (antibody hinge regions), and the structures of the antibody heavy chains were VH-CH1-Hinge-CH2-CH3, wherein the VH region can be further divided into FR1-CDR1-FR2-CDR2-FR3-CDR3-FR4 in detail. FR4 regions of the IgG2c antibody heavy chain variable regions produced in the IghM-3G3 homozygous mice were directly connected to the Hinge regions (the antibody hinge regions), indicating that the IgG2c of CH1 exons were successfully knocked out.

### Example 14. Phage display library construction and panning of CRP antigen-specific IgG2c heavy chain antibody variable regions

Following example 13 above, the steps for phage display library construction and antibody panning were exactly the same as those in example 6, only the parameters in Table 13 were adjusted, and the results obtained after 5 rounds of panning were as shown in Table 13 below:

**Table 13. CRP screening data sheet**

| Rounds | Coating amount of antigen | Amount of phages added | Times of number of PBST washing | Phage output | Enrichment degree |
|---|---|---|---|---|---|
| 1 | 10 µg | 2 × 10⁹ cfu | 3 | 2.4 × 10⁴ cfu | |
| 2 | 10 µg | 2 × 10¹⁰ cfu | 3 | 5 × 10⁶ cfu | 208.3 |
| 3 | 10 µg | 2 × 10¹¹ cfu | 10 | 1 × 10⁶ cfu | |
| 4 | 10 µg | 4 × 10¹¹ cfu | 10 | 3 × 10⁸ cfu | 300 |
| 5 | 10 µg | 2 × 10¹² cfu | 10 | 6 × 10⁸ cfu | 2 |

A total of 5 rounds of panning were performed; as can be seen from Table 13, the output phages were significantly enriched; and positive clones were selected by means of PHAGE-ELISA experiments and sent for sequencing identification, as shown in Table 14.

**Table 14: Amino acid sequences of positive clones**

| No. | Amino acid sequences |
|---|---|
| G3-23 | EVQLQQSGPEVLKPGTSVRMSCKASGYTFTDYNIHWVKQSRGESLEWIGFV NPDTGDTYYYKNFKGKATLTFNRSSSTAYMDLRSLTSEDSAVYYCARGTTVE KDYWGQGTILTVSS (SEQ ID NO: 47) |
| G3-35 | EVQLQQSGPELVKPGASVRMSCKASGYTFTDYNIHWVKQSHGESLEWIGFV |
| | NPDNGDTYYYKNFKGKATLTFNRSSSTAYMDLRSLTSEDSAVYYCARGTTVE KDYWGQGTTLTVSS (SEQ ID NO: 48) |
| G3-71 | EVQLQQSGPELVKPGASVRMSCKASGYTFTDYNIHWVKQSHGESLEWIGFI NPDNGDTYYYKNFRGKATLTLNKSSSTAYMDLRSLTSEDSGVYYCARGTTV EKDYWGQGTILTVSS (SEQ ID NO: 49) |

### Example 15. Antigen immunization and titer determination for IghM-3G3 homozygous mice

In this example, IghM-3G3 homozygous mice were immunized with a coronavirus S protein antigen.

An immunization method was as follows: 6- to 8-week-old male mice were selected, and the antigen coronavirus S protein (SARS-CoV-2 (2019-nCoV) Spike S1-His Recombinant Protein, 40591-V08H, Sino Biological Inc.) was emulsified with a freund's complete adjuvant (F5881, Sigma) with made a state of no dripping and then was used for subcutaneous multi-point injection for first immunization of mice, wherein the injected dose was 100 µg/mouse in the first immunization. After the first immunization, subsequent subcutaneous immunization was performed every 2 weeks, and the injected dose was 100 µg/mouse every time.

### Serum titer determination:

A method for determining a serum titer was as follows: a coronavirus S protein antigen was diluted to 2 µg/mL, 100 µL of diluted antigen was taken and added to a polystyrene enzyme-linked assay plate, the plate was covered, and a specific IgG antibody that specifically binds to the CRP antigen in serum was detected by using HRP-goat anti-mouse IgG-Fc (Jackson, 115-035-071).

Blood was collected when the mice was unimmunized and one week after the second immunization, one week after the third immunization and one week after the fourth immunization. The serum was subjected to doubling dilution with PBS, starting from a dilution degree of 1: 500. The serum titer was detected by ELISA, and the results were as shown in FIG. 17. As shown in FIG. 17, no specific antibody binding to the antigen coronavirus S protein exist in the unimmunized mice and after the second immunization, the serum titer thereof was about 1: 64000.

### Example 16. Extraction of IgG2c heavy chain antibody genes

According to example 15 above, the IghM-3G3 homozygous mice were immunized with an antigen coronavirus S protein and the serum titer was determined. Then, the mice were euthanized, splenocytes were taken and lysed with Trizol, total RNA was extracted and reversely transcribed to obtain cDNA, and a heavy chain variable region and a heavy chain constant region connected thereto were amplified by PCR by using the following IgG2c isotype antibody-specific primers.

See example 5 for MHV1, MHV2, MHV3, MHV4, MHV5, MHV6, MHV7, MHV8, MHV9, MHV10, MHV11, MHV12 and B6_IgG2c_CH2 R1.

The PCR reaction system and the PCR reaction process were as shown in example 5.

The obtained PCR amplification product was connected with a pEASY-T5 Zero Cloning vector, transformed TOP10 competent cells and coated an LB plate. 23 clones were randomly selected, and the clones identified as positive by means of colony PCR were sent for sequencing (PCR results were as shown in FIG. 18A, wherein the band of 650 bp was a positive band).

### Example 17. Phage display library construction and panning of coronavirus S protein antigen-specific IgG2c heavy chain antibody variable region

Following example 16 above, the steps for phage display library construction and antibody panning were exactly the same as those in example 6. A total of 5 rounds of panning were performed; the output phages were significantly enriched every time; and positive clones were selected by means of PHAGE-ELISA experiments and sent for sequencing identification, as shown in Table 15. The similarities and differences of the CDR regions of these sequences were analyzed, and the results were as shown in FIG. 18B.

**Table 15: Amino acid sequences of positive clones**

| No. | Amino acid sequences |
|---|---|
| S-9 | EVQLQQSGPELVKPGASVKISCKASGYTFTDYYMNWVKQSHGKSLEWIGDI NPNNGDANYNQSFKGKATLTVDKSSSTAYMELRSLTSEDSAVYYCAKPPLYY YNDSHWYFNVWGTGTTVTVSS (SEQ ID NO: 50) |
| S-19 | EVQLQQSGPELVKPGASVKISCKASGYTFTDYYMNWVKQSHGKSLEWIGDI NPNNSDSNYNQNFKDKATLTVDRSSSTAYMELRSLTSEDSAVYYCAKPPLYY YNNSHWYFNVWGTGTTVTVSS (SEQ ID NO: 51) |
| S-27 | EVQLQQSGPELVKPGASVKISCKASGYTFTDYYMNWVKQSHGRSLEWIGDI NPNNGDTNYNPNFKDKATLTVDMSSTTAYMELRSLTSEDSAVYFCAKPPLYY YNNSHWYFNVWGTGTTVTVSS (SEQ ID NO: 52) |
| S-47 | EVQLQQSGPELVKPGDSVKISCKASGYSFTDYFINWVMRSHGKGLEWIGRIN PYNGDTFFNQKFKGKAILTVDTSSSTAHMELRSLTSEDSAVYYCATYGSPMD YWGQGTSVTVSS (SEQ ID NO: 53) |

The sequences for sequencing were analyzed, and the S-9, S-19, S-27 and S-47 (amino acid sequences were as shown in Table 15) that appeared in the first 5 rounds of panning were selected to transfect BL21 Escherichia coli. Different concentrations of IPTG were added at 30°C, prokaryotic expression was induced, ultrasonication was performed, and the supernatant was collected and purified by means of a nickel affinity column.

A specific antigen coronavirus S protein and an unrelated protein OVA (2 µg/mL) were coated, and the purified S-9, S-19, S-27 and S-47 antibodies were subjected to gradient dilution and detected by means of ELISA. The results showed that the variable region of the heavy chain antibody extracted this time can specifically recognize and bind to the antigen coronavirus S protein, and showed a good concentration dependence, as shown in FIG. 19.

### Example 18. Antigen immunization and titer determination for IghM-DG1 homozygous mice

In this example, IghM-DG1 homozygous mice were immunized with a coronavirus S protein antigen.

The mouse antigen immunization operation and the step for serum titer determination were exactly the same as those in example 15, and the results of the serum titer determination were as shown in FIG. 20A. The steps for phage display library construction and antibody panning were exactly the same as those in example 6. A total of 5 rounds of panning were performed; the output phages were significantly enriched every time; and positive clones were selected by means of PHAGE-ELISA experiments and sent for sequencing identification, as shown in Table 16. The similarities and differences of the CDR regions of these sequences were analyzed, and the results were as shown in FIG. 20B.

**Table 16: Amino acid sequences of positive clones**

| No. | Amino acid sequences |
|---|---|
| S-1 | EVQLQQSGPELVKPGASVKISCKASGYTFTDYYMNWVKQSHGKSLEWIGDI NPNNGGANYNQTFKGKATFTVDKSSSTAYMELRSLTSEDSAVYYCAKGPLY YYNSSNWYFDVRGTGTTVTVSS (SEQ ID NO: 54) |
| S-2 | EVQLQQSGPELVKPGASVKISCKASGYTFTDYYMNWVKQSHGKSLEWIGDI NPNNGGTSYNQKFKGKATLTVDKSSSTAYMELRSLTSEDSAVYYCAKGPLYY YNSSNWYFDVRGTGTTVTVSS (SEQ ID NO: 55) |
| S-7 | EVQLQQSGPELVKPGASVKISCKASGYTFTDYYINWVKQSHGKSREWIGDIN PKNGGTNFNREFKGEATLTVDKSSTTAYMELRSLTSEDSAVYYCAKGPLYYY NNSYWYFDVWGTGTTVTVSS (SEQ ID NO: 56) |
| S-12 | EVQLQQSGPELVKPGASVKISCKASGYTFTDYYMNWVKQSHGKSREWIGDI |
| | NPNNGVTSYNQNFKGEATLTVDKSSSTAYMELRSLTSEDSAVYYCAKGPLYY YNSSNWYFDVWGTGTTVTVSS (SEQ ID NO: 57) |
| S-17 | EVQLQQSGPELVKPGASVKISCKASGYMFTDYYMNWVKQSHGKSLEWIGDI NPNNGGANYNQKFKGKATLTVDKSSSTAYMELRSLTSEDSAVYYCAKGPLY YYNSSNWYFDVRGTGTTVTVSS (SEQ ID NO: 58) |
| S-19 | EVQLQQSGPELVKPGASVKISCKASGYTFTDYYMNWVKQSHGKSLEWIGDI NPNNGGANYNQTFKGKATLTVDKSSSTAYMELRSLTSEDSAVYYCAKGPLY YYNSSTWYFDVRGTGTTVTVSS (SEQ ID NO: 59) |
| S-25 | EVQLQQSGPELVKPGASVKISCKASGYTFTDYYMNWVKQSHGKSLEWIGDI NPNNGGTSYNQKFKGKATLTVDKSSSTAYMELRSLTSEDSAVYYCAKGPLYY YNNSNWYFDVRGTGTTVTVSS (SEQ ID NO: 60) |
| S-51 | EVQLQQSGPELVKPGASVKISCKASGYTFTDYYMNWVKQSHGKSLEWIGDI NPSTGGTSYNQTFKGKATLTVDKSSSTAYMELRSLTSEDSAVYYCAKGPLYY YNSSNWYFDVRGTGTTVTVSS (SEQ ID NO: 61) |
| S-65 | EVQLQQSGPELVKPGASVKISCKASGYTFTDYYINWVKQSHGKSLEWIGDIN PKNGGSNYNQTFKGEATLTVDKSSNTAYMELRSLTSEDSAVYYCARGPLYYY NSTYWYFDVWGTGTTVTVSS (SEQ ID NO: 62) |

The sequences for sequencing were analyzed, and DNA sequences of the S-1, S-2, S-7, S-12, S-17, S19 and S-65 antibodies (amino acid sequences were as shown in table 16) that appeared in the first 5 rounds of panning were selected, cloned into a PET28 vector and transfected BL21 Escherichia coli. Different concentrations of IPTG were added at 16°C, prokaryotic expression was induced, ultrasonication was performed, and the supernatant was collected and purified by means of a nickel affinity column.

A specific antigen coronavirus S protein and an unrelated protein OVA (2 µg/mL) were coated, and the purified S-1, S-7, S-12, S-17, S19, S-25, S-51 and S-65 antibodies were subjected to gradient dilution and detected by means of ELISA. The results showed that the variable region of the heavy chain antibody extracted this time can specifically recognize and bind to the antigen coronavirus S protein, and showed a good concentration dependence, as shown in FIG. 21.

### Example 19. Extraction of IgG2c heavy chain antibody genes

In this example, MDG1 homozygous mice were immunized with an antigen coronavirus N protein.

The immunization method and the method for serum titer determination were exactly the same as those in example 15, and after immunization, the results of the serum titer determination were as shown in FIG. 22A. The antigen used in this example was coronavirus N protein (SARS-CoV-2 (2019-nCoV) Nucleocapsid-His recombinant Protein, 40588-V08B-B, Sino Biological Inc.).

The steps for phage display library construction and antibody panning were exactly the same as those in example 6. A total of 5 rounds of panning were performed; the output phages were significantly enriched every time; and positive clones were selected by means of PHAGE-ELISA experiments and sent for sequencing identification, as shown in Table 17. The similarities and differences of the CDR regions of these sequences were analyzed, as shown in FIG. 22B.

**Table 17: Amino acid sequences of positive clones**

| No. | Amino acid sequences |
|---|---|
| N-1 | EVQLQESGPGMVKPSQSLSLTCTVTGYSITSGYDWHWIRQFPGNKQEWLGY ITYDGSNHYNPSLKNRISITRDTSKNQFFLKLSSVTTEDTATYYCARGDDYDG DYYTMDYWGQGTSVTVSS (SEQ ID NO: 63) |
| N-2 | EVQLKESGGGLVKPGGSLKLSCAASGFTFSDYGMHWVRQAPEKGLEWVAYI SSGSSTIYYADTVKGRFTISRDNAKNTLFLQMTSLRSEDTAIYYCARLDDYDA DYYAMDYWGQGTSVTVSS (SEQ ID NO: 64) |
| N-3 | QVQLKESGPGLVKPSQSLSLTCTVTGYSITSGYDWHWIRHFPGNKLEWMGYI SYSGSTNYNPSLKSRISITHDTSKNHFFLKLNSVTTEDTATYYCARGDDYDGD YYTMDYWGQGTSVTVSS (SEQ ID NO: 65) |
| N-5 | EVQLKESGPGLVKPSQSLSLTCSVTGYSITSGYYWYWIRQFPGNKQEWLGYI TYDGSNHYNPSLKNRISITRDTSKNQFFLKLSSVTTEDTATYYCARGDDYDG DYYTMDYWGQGTSVTVSS (SEQ ID NO: 66) |
| N-23 | EVQLQESGGGLVKPGGSLKLSCAASGFTFSDYGMHWVRQAPEKGLEWVAYI SSGSSTIYYADTVKGRFTISRDNAKNTLFLQMTSLRSEDTAMYYCARGLDAM DNWGQGTSVTVSS (SEQ ID NO: 67) |

The DNA sequences of the positive clones were cloned into a PET28 vector and transfected BL21 bacteria, induced expression was performed, then a target protein was purified by using a nickel column, and the specificity and binding ability of the antibodies were detected by means of ELISA. The results were as shown in FIG. 23. As shown in FIG. 23, the results showed that the variable region of the heavy chain antibody extracted this time can specifically recognize and bind to the antigen coronavirus S protein, and showed a good concentration dependence.

### Example 20. Differences of gene transcription levels of immunoglobulin genes in mice of two genotypes as detected by fluorescent quantitative PCR detection

The spleens of MDG1 mice and wild-type mice were taken, and RNA was extracted and reversely transcribed into cDNA. The cDNA was subjected to 5-fold dilution, and then 5 µL of diluent was taken for a fluorescent quantitative PCR experiment. Four individuals were selected for each genotype, and each gene of each individual was subjected to 2 technical repetitions. The lengths of primers and amplified fragments used in the experiment were as shown in Table 18, and the fluorescent quantitative PCR amplification system was as shown in Table 19.

**Table 18: Lengths of primer sequences and amplification products used in fluorescent quantitative PCR experiment**

| Primers | Sequences | Lengths of PCR products |
|---|---|---|
| JH2 | 5'-CTACTGGGGCCAAGGCAC-3' (SEQ ID NO: 68) | µ: 119 bp |
| q-IgM-CH1-R | 5'-AGCCCATGGCCACCAGATTC-3' (SEQ ID NO: 69) | |
| q-IgG2c-hinge-CH2-F | 5'-ATGCGCAGCTCCAGACCTCTTG-3' (SEQ ID NO: 70) | y2c: 86 bp |
| q-IgG2c-CH2-R | 5'-GGCTCAGGGAGATCATGAG-3' (SEQ ID NO: 71) | |
| JH2-3 | 5'-TACTGGGGCCAAGGCACCACTC-3' (SEQ ID NO: 72) | α: 98 bp |
| q-IgA-CH1-R | 5'-GGGTCACTTGACAGAGCTCGTG-3' (SEQ ID NO: 73) | |

**Table 19: Fluorescent quantitative PCR amplification system**

| | |
|---|---|
| 2 × SYBR Green mix | 10 µL |
| Upstream primer (10 µM) | 1 µL |
| Downstream primer (10 µM) | 1 µL |
| cDNA | 5 µL |
| ddH₂O | 3 µL |

### Amplification process:

| | |
|---|---|
| 95°C: 15 min | 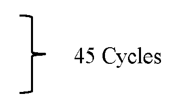 |
| 95°C: 10 s | |
| 60°C: 20 s | |
| 72°C: 20 s | |

### Dissolution curve

### 65°C: 1 min

Continuously raise the temperature to 95°C at a rate of 0.11°C/s

After the process was completed, the degree of credibility of a numerical value was judged according to the dissolution curve, then the relative expression level of each immunoglobulin gene transcript was calculated by using a 2^{-ΔΔCt} method, and statistical analysis was performed by using a two-tailed T test. The statistical results were as shown in FIG. 24, FIG. 25 and FIG. 26. As can be seen from these results: the expression quantity of transcripts of µ genes in the bone marrow, spleens and small intestines of MDG1 mice was lower than that of wild-type mice, and the transcript expression quantities of y2c genes were higher than those of wild-type mice; and the expression quantity of transcripts of y2c genes in the three pieces of immune tissue of MDG1 mice was higher than the expression quantity of transcripts of µ genes in wild-type mice, and the expression quantity of transcripts of α genes was not statistically significant in the mice of two genotypes.

### Example 21. Expression form of IgG2c in serum of MDG1 mice as detected by Western blot

The serum of 8-week-old wild-type mice and MDG1 mice under the same genetic background were taken for Western Blot detection. The serum of the wild-type mice was subjected to 20-fold dilution with PBS, and the serum of the MDG1 mice was subjected to 100-fold dilution with PBS. Under reducing conditions, an intramolecular disulfide bond was opened by using 1mM of DTT, and the Goat Anti-Mouse IgG2c heavy chain (HRP) antibody was subjected to 10000-fold dilution. The results of Western Blot detection were as shown in FIG. 27. As can be seen from FIG. 27, IgG2c in the serum of the MDG1 mice had a molecular weight of about 45 kDa under reducing conditions and about 95 kDa under non-reducing conditions, which is consistent with the molecular weights lacked by the CH1 domain and the light chain deletion.

### Example 22. Expression quantities of immunoglobulin in serum of MDG1 mice as detected by ELISA

The serum of 8-week-old wild-type mice and MDG1 mice under the same genetic background were detected by using double-antibody sandwich ELISA. For the wild-type mice, IgM was subjected to 2000-fold dilution, IgG2c was subjected to 4000-fold dilution, IgG was subjected to 10000-fold dilution, IgA was subjected to 5000-fold dilution, and IgE was subjected to 20-fold dilution; and for the MDG1 mice, IgM was subjected to 2000-fold dilution, IgG2c was subjected to 8000-fold dilution, IgG was subjected to 10000-fold dilution, IgA was subjected to 5000-fold dilution, and IgE was subjected to 20-fold dilution. The absorbance value of each sample well at 450 nm was read by using a microplate reader, a standard curve was obtained by using ELISA Calc according to four-parameter fitting, the content of immunoglobulin in each well was calculated, and statistical analysis was performed by using a two-tailed T test.

The results were as shown in FIG. 28. As can be seen from FIG. 28, IgM was not expressed in the serum of the MDG1 mice, and the expression level of IgG2c of the MDG1 mice was significantly higher than that of wild-type mice and comparable to the expression level of IgM in wild-type mice. Since there were other isotypes of IgG in the wild-type mice, the expression level of total IgG in the serum of the wild-type mice was significantly higher than that of the MDG1 mice, the expression level of IgE in the serum of the MDG1 mice was significantly lower than that of the wild-type mice, and there was no statistical difference between the expression levels of IgA of both the MDG1 mice and the wild-type mice.

### Example 23. Growth of B cells of bone marrow, spleens and peritoneal cavities of MDG1 mice as detected by flow cytometry

### B cells of bone marrow:

Six 8-week-old wild-type mice and six 8-week-old MDG1 mice under the same genetic background were prepared. Two femurs and tibiae of the lower limbs of each mouse were taken, and bone marrow cells were flushed into a centrifuge tube with FACS, centrifuged transiently and then resuspended with ACK. The cell suspension was filtered with a 70-µm filter screen and centrifuged, and then the supernatant was discarded. The cells were resuspended by adding 1 mL of FACS, 50 µL of the resuspended cells was taken and stained, and 10 µL of stained cells was taken, subjected to 40-fold dilution and calculated. A staining solution for the B cells of the bone marrow was as shown in table 20.

### B cells of spleens:

Four 8-week-old wild-type mice and four 8-week-old MDG1 mice under the same genetic background were prepared. A 70-µm filter screen was placed in a 60 mm dish, 1 mL of ACK was added, and the spleens of the mice were gently ground on the filter screen. The cell suspension obtained after grinding was collected and centrifuged transiently, and the supernatant was discarded. The cells were resuspended by adding 1 mL of FACS, 50 µL of the resuspended cells was taken and stained, and 10 µL of stained cells was taken, subjected to 40-fold dilution and calculated. A staining solution for the B cells of the spleens was as shown in Table 20.

### B cells of peritoneal cavities:

Seven 8-week-old wild-type mice and seven 8-week-old 7 MDG1 mice under the same genetic background were prepared, and the abdomens of the mice were fixed upward to a foam board, and the ventral epidermis was cut to expose the peritoneum part. 5 mL of FACS was aspirated with a syringe into the peritoneal cavity, blowing-suction was performed 2-3 times by using a pipette and the cell suspension was collected into a centrifuge tube, centrifuged transiently, then resuspended by using ACK, filtered with a 70-µm filter screen and centrifuged, and then the supernatant was discarded. The cells were resuspended by adding 200 µL of FACS, and 100 µL of the resuspended cells was taken and stained. A staining solution for the B cells of the peritoneal cavities was as shown in Table 20.

**Table 20: Flow cytometry staining solution**

| | APC | V450 | V500 | PE | PE-Cy7 | PerCP | APC-Cy7 | FITC |
|---|---|---|---|---|---|---|---|---|
| Bone marrow | | B220 | | CD43 | IgM | | | IgG2c |
| Spleen | B220 | CD138 | CD19 | CD23 | CD21 | 7-AAD | IgM | IgG2c |
| Peritoneal cavity | CD23 | | | CD5 | CD19 | 7-AAD | | |

The flow cytometry results of B cells in the bone marrow were as shown in FIG. 29. As shown in FIG. 29, the results showed that the proportion and number of B cells in the bone marrow of the MDG1 mice had no statistical difference in comparison with the wild-type mice, and the proportions of the pro-B cells and the pre-B cells had no statistical difference, but the numbers of the pro-B cells and the pre-B cells were significantly reduced in comparison with wild-type mice; IgM was highly expressed in mature B cells and low expressed in immature B cells; and since the MDG1 mice lacked µ genes expressing IgM, it was impossible to determine the growth conditions of immature B cells and mature B cells in the body, but a large number of IgG2c + B cells existed in MDG1 mice, whereas the proportion of these B cells was extremely low in the wild-type mice.

The flow cytometry results of B cells in the spleens were as shown in FIG. 30. As shown in FIG. 30, the results showed that the proportion of B cells in the spleens of the MDG1 mice was significantly lower than that of wild-type mice, but there was no statistical difference in the number; the proportion and number of IgG2c + B cells in the spleens of the MDG1 mice were significantly higher than those of wild-type mice, which may result from that IgM in the wild-type mice was subjected to class switch and recombined to other isotypes of immunoglobulin; since this flow cytometry experiment selected mice without immunostimulation, the proportion and number of plasma cells in the spleens of the wild-type mice and the MDG1 mice were both lower, and no statistical difference was observed; and the proportions and numbers of follicular B cells, transitional B cells and marginal zone B cells in the spleens of the MDG1 mice do not have statistical difference in comparison with the wild-type mice.

The flow cytometry results of B cells in the peritoneal cavities were as shown in FIG. 31. As shown in FIG. 31, the results showed that the proportion of B cells in the peritoneal cavities of the MDG1 mice was significantly higher than that of the wild-type mice, and the proportion of IgM + B1a cells was significantly reduced in the MDG1 mice, whereas the proportions of B1b and B2 cells were significantly increased in the MDG1 mice.

### Example 24. Immunostimulation of wild-type mice and MDG1 mice with specific antigens

Six to seven 6-week-old wild-type and six to seven 6-week-old MDG1 mice under the same genetic background were prepared, and immunization experiments were performed by using chloramphenicol and atrazine coupled with BSA, 100 µg/100 µL per mouse per immunization. A Freund's complete adjuvant was used during the first immunization, wherein 50 µL of Freund's complete adjuvant was injected subcutaneously in the back and 50 µL of Freund's complete adjuvant was injected intraperitoneally; a Freund's incomplete adjuvant was used during the booster immunizations, wherein 100 µL of Freund's incomplete adjuvant was injected intraperitoneally, and a total of four booster immunizations were performed; and 3 days before the first immunization and 3 days after each booster immunization, blood was collected from the inner canthus.

### Example 25. Change trend of relative expression quantity of antigen-specific antibody in serum of mice after immunostimulation as detected by ELISA

Following example 24 above, in order to avoid the interference with the detection results caused by a large amount of BSA-specific antibodies in the serum after immunostimulation, this ELISA experiment used chloramphenicol and atrazine coupled with OVA as coating antigens. The coating amount of each antigen was 200 ng/100 µL/well, the serum was subjected to 4000-fold dilution in the detection of antigen-specific IgM, IgG2c, IgG and IgA, and the serum was subjected to 100-fold dilution in the detection of IgE. In order to ensure the consistency of each sample well, a TMB chromogenic solution was added, then the reaction time was strictly controlled to 20 min, then the absorbance value at 450 nm was read with a microplate reader, the individuals without immunization were excluded, and a diagram of the change trend of the relative expression quantity of the antigen-specific antibody was plotted.

The changes in the relative expression quantities of specific antibodies chloramphenicol and atrazine were as shown in FIG. 32 and FIG. 33, respectively. The results showed that: from the perspective of the overall immune response, the immunization effect of chloramphenicol was better than that of atrazine. The change trend of antigen-specific IgG2c in the MDG1 mice was similar to that of IgG2c in the wild-type mice, which is different from the continuous low-level expression of IgM in the wild-type mice. Since there were other isotypes of IgG in the wild-type mice, the content of antigen-specific total IgG in the wild-type mice was higher than that in the MDG1 mice, and in addition, the relative expression quantities of antigen-specific IgA and IgE in the MDG1 mice was slightly higher than those in the wild-type mice.

### Example 26. Detection of growth of germinal center B cells and plasma cells in the spleens after antigen immunization

Six wild-type mice and six MDG1 mice obtained after antigen immunization were taken. A 70-µm filter screen was placed in a 60 mm dish, 1 mL of ACK was added, and the spleens of the mice were gently ground on the filter screen. The cell suspension obtained after grinding was collected and centrifuged transiently, and the supernatant was discarded. The cells were resuspended by adding 1 mL of FACS, 20 µL of the resuspended cells was taken and stained, and 10 µL of stained cells was taken, subjected to 80-fold dilution and calculated. A staining solution for the B cells of the spleens obtained after immunization was as shown in Table 21.

**Table 21: Staining solution for spleen B cells after immunization**

| | FITC | PerCP | APC | PE-Cy7 | APC-Cy7 | V450 | V500 |
|---|---|---|---|---|---|---|---|
| Spleen | IgG2c | 7-AAD | B220 | Fas | CD38 | CD138 | CD19 |

The growth conditions of spleen B cells, germinal center B cells and plasma cells in the mice obtained after antigen immunization were as shown in FIG. 34. As shown in FIG. 34, the results showed that the proportion of spleen B cells of the MDG1 mice obtained after immunization was significantly lower than that of the wild-type mice, but there was no statistical difference in the number; the proportion and number of germinal center B cells in the MDG1 mice were significantly lower than those in the wild-type mice, and the proportion of IgG2c + GC B cells in the MDG1 mice was significantly higher than that in wild-type mice, but there was no statistical difference in the number; and there was no statistical difference in the proportions and numbers of plasma cells and IgG2c + plasma cells of the MDG1 mice and the wild-type mice after antigen immunization.

Finally, it should be noted that: the above examples are only used to illustrate the technical solution of the present application, rather than to limit them. Although the present application was described in detail with reference to the foregoing examples, a person skilled in the art should understand that it was still possible to amend the technical solutions described in the foregoing examples or to make equivalent replacements to some of the technical features thereof; and these amendments or replacements should not make the essence of the corresponding technical solutions deviate from the spirit and scope of the technical solutions of the examples of the present application.

### INDUSTRIAL PRACTICABILITY

The non-human mammal or offspring thereof obtained by using the method for preparing the non-human mammal or offspring thereof in the present application does not introduce any exogenous gene encoding heavy chain variable and constant regions of an antibody, and can directly use all VDJ genes encoding the heavy chain variable region of the antibody in its own genome, so as to produce more diverse heavy chain antibodies by means of the rearrangement of the heavy chain variable region.

## Claims

1. A method for preparing a non-human mammal or progeny thereof, wherein the method comprises the following steps:
making a CH1 domain of an IgM heavy chain constant region not expressed or incorrectly expressed in the non-human mammal; and
making one, two, three, four or more genes encoding an IgG heavy chain constant region in the non-human mammal not express or incorrectly express the CH1 domain during expression.

2. The preparation method according to claim 1, wherein making the CH1 domain of the IgM heavy chain constant region not expressed or incorrectly expressed in the non-human mammal is, only making the CH1 domain of the IgM heavy chain constant region not expressed or incorrectly expressed in the non-human mammal, or
making the IgM heavy chain constant region and an IgD heavy chain constant region not expressed or incorrectly expressed in the non-human mammal.

3. The preparation method according to claim 1 or 2, wherein making one, two, three, four or more genes encoding the IgG heavy chain constant region in the non-human mammal not express or incorrectly express the CH1 domain during expression is,
making a first gene encoding the IgG heavy chain constant region in the non-human mammal not express or incorrectly express the CH1 domain during expression; or
making the first gene encoding the IgG heavy chain constant region in the non-human mammal not express or incorrectly express the encoded IgG heavy chain constant region during expression, and making one, two or three of a second or a third or a fourth gene encoding the IgG heavy chain constant region in the non-human mammal not express or incorrectly express the CH1 domain; or making the first and second genes encoding the IgG heavy chain constant region in the non-human mammal not express or incorrectly express the encoded IgG heavy chain constant region during expression, and making one or two of the third or fourth genes encoding the IgG heavy chain constant region in the non-human mammal not express or incorrectly express the CH1 domain; or making the first, second and third genes encoding the IgG heavy chain constant region in the non-human mammal not express or incorrectly express the encoded IgG heavy chain constant region during expression, and making the fourth gene encoding the IgG heavy chain constant region in the non-human mammal not express or incorrectly express the CH1 domain during expression; or
making the first gene encoding the IgG heavy chain constant region in the non-human mammal correctly express the encoded IgG heavy chain constant region during expression, and making one, two or three of the second or third or fourth genes encoding the IgG heavy chain constant region in the non-human mammal not express or incorrectly express the CH1 domain; or
making the first and second genes encoding the IgG heavy chain constant region in the non-human mammal correctly express the encoded IgG heavy chain constant region during expression, and making one or two of the third or fourth genes encoding the IgG heavy chain constant region in the non-human mammal not express or incorrectly express the CH1 domain; or
making the first, second and third genes encoding the IgG heavy chain constant region in the non-human mammal correctly express the encoded IgG heavy chain constant region during expression, and making the fourth gene encoding the IgG heavy chain constant region in the non-human mammal not express or incorrectly express the CH1 domain during expression.

4. A method for preparing a non-human mammal or progeny thereof, wherein the method comprises the following steps:
knocking out a nucleotide sequence, comprising a nucleotide sequence encoding a CH1 domain of an IgM heavy chain constant region, on a genome of the non-human mammal; and
knocking out a target gene, comprising nucleotide sequence(s) encoding CH1 domain(s) on one, two, three, four or more genes encoding an IgG heavy chain constant region.

5. The preparation method according to claim 4, wherein knocking out a nucleotide sequence, comprising a nucleotide sequence encoding the CH1 domain of the IgM heavy chain constant region, on the genome of the non-human mammal is
only knocking out the nucleotide sequence encoding the CH1 domain of the IgM heavy chain constant region on the genome of the non-human mammal; or
knocking out a nucleotide sequence encoding the IgM heavy chain constant region and a nucleotide sequence encoding an IgD heavy chain constant region on the genome of the non-human mammal.

6. The preparation method according to claim 4 or 5, wherein the target gene is the nucleotide sequence, encoding the CH1 domain on a first gene encoding the IgG heavy chain constant region; or
all nucleotide sequences, encoding the CH1 domain, on a region starting from the first gene encoding the IgG heavy chain constant region to a second gene encoding the IgG heavy chain constant region; or
all nucleotide sequences, encoding the CH1 domain, on the region starting from the first gene encoding the IgG heavy chain constant region to a third gene encoding the IgG heavy chain constant region; or
all nucleotide sequences, encoding the CH1 domain, on the region starting from the first gene encoding the IgG heavy chain constant region to a fourth gene encoding the IgG heavy chain constant region; or
all nucleotide sequences, encoding the CH1 domain, on the region starting from the first gene encoding the IgG heavy chain constant region to a last gene encoding the IgG heavy chain constant region; or
the nucleotide sequence, encoding the CH1 domain, on the second gene encoding the IgG heavy chain constant region; or
all nucleotide sequences, encoding the CH1 domain, on the region starting from the second gene encoding the IgG heavy chain constant region to the third gene encoding the IgG heavy chain constant region; or
all nucleotide sequences, encoding the CH1 domain, on the region starting from the second gene encoding the IgG heavy chain constant region to the fourth gene encoding the IgG heavy chain constant region; or
the nucleotide sequence, encoding the CH1 domain, on the third gene encoding the IgG heavy chain constant region; or
all nucleotide sequences, encoding the CH1 domain, on the region starting from the third gene encoding the IgG heavy chain constant region to the fourth gene encoding the IgG heavy chain constant region; or
the nucleotide sequence, encoding the CH1 domain, on the fourth gene encoding the IgG heavy chain constant region.

7. The preparation method according to any one of claims 4-6, wherein the following steps are completed in a same operation step or in different operation steps:
knocking out a nucleotide sequence, comprising a nucleotide sequence encoding the CH1 domain of the IgM heavy chain constant region, on the genome of the non-human mammal; and
knocking out a target gene.

8. The preparation method according to any one of claims 1-7, wherein the non-human mammal is a rodent; optionally, the rodent is a rat or a mouse; further optionally, the rodent is a mouse; and still further, the mouse is a C57BL/6 mouse or a BALB/c mouse.

9. The preparation method according to claim 3, 6, 7 or 8, wherein the first gene encoding the IgG heavy chain constant region is Ighg3.

10. The preparation method according to claim 3, 6, 7, 8 or 9, wherein when the non-human mammal is the C57BL/6 mouse, the first gene encoding the IgG heavy chain constant region is Ighg3, the second gene encoding the IgG heavy chain constant region is Ighg1, the third gene encoding the IgG heavy chain constant region is Ighg2b, and the fourth gene encoding the IgG heavy chain constant region is Ighg2c; and
when the non-human mammal is the BALB/c mouse, the first gene encoding the IgG heavy chain constant region is Ighg3, the second gene encoding the IgG heavy chain constant region is Ighg1, the third gene encoding the IgG heavy chain constant region is Ighg2b, and the fourth gene encoding the IgG heavy chain constant region is Ighg2a.

11. The preparation method according to any one of claims 1-10, wherein the genome of the non-human mammal comprises a complete gene encoding a κ light chain and/or a λ light chain; and optionally, the κ light chain and/or the λ light chain can be normally expressed in the non-human mammal.

12. The preparation method according to any one of claims 1-10, wherein the gene knockout method comprises one or more of followings: a gene targeting technology, a CRISPR/Cas9 method, a zinc finger nuclease method and a transcription activator-like effector nuclease method.

13. The preparation method according to any one of claims 1-10, wherein the non-human mammal or progeny thereof is used for preparing a heavy chain antibody.

14. A method for preparing a C57BL/6 mouse or progeny thereof, wherein the method comprises following steps:
knocking out a gene, encoding an antibody IgM heavy chain constant region and an antibody IgD heavy chain constant region, in a genome of the C57BL/6 mouse; and
knocking out a nucleotide sequence, encoding a CH1 domain, on a region starting from the gene encoding an IgG3 heavy chain constant region to the gene encoding an IgG2c heavy chain constant region in the genome of the C57BL/6 mouse.

15. The preparation method according to claim 14, wherein the genome of the C57BL/6 mouse comprises a complete gene encoding a κ light chain and/or an λ light chain; and optionally, the C57BL/6 mouse can normally express the κ light chain and/or the λ light chain.

16. The preparation method according to claim 14, wherein the gene knockout method comprises one or more of followings: a gene targeting technology, a CRISPR/Cas9 method, a zinc finger nuclease method and a transcription activator-like effector nuclease method.

17. The preparation method according to claim 14, wherein in the gene knockout step, all nucleotide sequences from the first exon of the gene encoding the IgM heavy chain constant region to the first exon of the gene encoding the IgG2c heavy chain constant region in the mouse are knocked out.

18. The preparation method according to claim 17, wherein in the gene knockout step, sgRNA targeting upstream of the first exon of the gene encoding the IgM heavy chain constant region, and sgRNA targeting downstream of the first exon of the gene encoding the IgG2c heavy chain constant region are used;
optionally, a targeting sequence, targeted by sgRNA, on the upstream of the first exon of the gene encoding the IgM heavy chain constant region in the mouse comprises SEQ ID NO. 1 and SEQ ID NO. 2; and/or the targeting sequence, targeted by sgRNA, on the downstream of the first exon of the gene encoding the IgG2c heavy chain constant region in the mouse comprises SEQ ID NO. 3 and SEQ ID NO. 4.

19. The preparation method according to claim 18, wherein sgRNA targeting the upstream of the first exon of the gene encoding the IgM heavy chain constant region in the mouse is SEQ ID NO. 9 and SEQ ID NO. 10; and/or sgRNA targeting the downstream of the first exon of the gene encoding the IgG2c heavy chain constant region in the mouse is SEQ ID NO. 11 and SEQ ID NO. 12.

20. The preparation method according to any one of claims 14-19, wherein the C57BL/6 mouse or progeny thereof is used for preparing a heavy chain IgG2c antibody.

21. A method for preparing a C57BL/6 mouse or progeny thereof, wherein the method comprises following steps:
knocking out a nucleotide sequence, encoding a CH1 domain, on a gene encoding an IgM heavy chain constant region in a genome of the C57BL/6 mouse; and
knocking out nucleotide sequences, encoding the CH1 domain, on a region starting from the gene encoding an IgG3 heavy chain constant region to the gene encoding an IgG2c heavy chain constant region in the genome of the C57BL/6 mouse.

22. The preparation method according to claim 21, wherein the genome of the C57BL/6 mouse comprises a complete gene encoding a κ light chain and/or an λ light chain; and optionally, the C57BL/6 mouse can normally express the κ light chain and/or the λ light chain.

23. The preparation method according to claim 22, wherein the gene knockout method comprises one or more of followings: a gene targeting technology, a CRISPR/Cas9 method, a zinc finger nuclease method and a transcription activator-like effector nuclease method.

24. The preparation method according to claim 21, wherein in the gene knockout step, the first exon of the gene encoding the IgM heavy chain constant region in the mouse is knocked out, and all nucleotide sequences from the first exon of the gene encoding the IgG3 heavy chain constant region to the first exon of the gene encoding the IgG2c heavy chain constant region in the mouse are further knocked out.

25. The preparation method according to claim 24, wherein in the gene knockout step, sgRNA targeting the upstream and downstream of the first exon of the gene encoding the IgM heavy chain constant region in the mouse is used, and sgRNA targeting the upstream of the first exon of the gene encoding the IgG3 heavy chain constant region in the mouse and sgRNA targeting the downstream of the first exon of the gene encoding the IgG2c heavy chain constant region in the mouse are further used.

26. The preparation method according to claim 25, wherein a targeting sequence, targeted by sgRNA, on the upstream of the first exon of the gene encoding the IgM heavy chain constant region in the mouse comprises SEQ ID NO. 1 and SEQ ID NO. 2; and/or
the targeting sequence, targeted by sgRNA, on the downstream of the first exon of the gene encoding the IgM heavy chain constant region in the mouse comprises SEQ ID NO. 5 and SEQ ID NO. 6; and/or
the targeting sequence, targeted by sgRNA, on the upstream of the first exon of the gene encoding the IgG3 heavy chain constant region in the mouse comprises SEQ ID NO. 7 and SEQ ID NO. 8; and/or
the targeting sequence, targeted by sgRNA, on the downstream of the first exon of the gene encoding the IgG2c heavy chain constant region in the mouse comprises SEQ ID NO. 3 and SEQ ID NO. 4.

27. The preparation method according to claim 26, wherein sgRNA targeting the upstream of the first exon of the gene encoding the IgM heavy chain constant region in the mouse is SEQ ID NO. 9 and SEQ ID NO. 10; and/or
sgRNA targeting the downstream of the first exon of the gene encoding the IgM heavy chain constant region in the mouse is SEQ ID NO. 13 and SEQ ID NO. 14; and/or
sgRNA targeting the upstream of the first exon of the gene encoding the IgG3 heavy chain constant region in the mouse is SEQ ID NO. 15 and SEQ ID NO. 16; and/or
sgRNA targeting the downstream of the first exon of the gene encoding the IgG2c heavy chain constant region in the mouse is SEQ ID NO. 11 and SEQ ID NO. 12.

28. The preparation method according to any one of claims 20-27, wherein the C57BL/6 mouse or progeny thereof is used for preparing a heavy chain IgG2c antibody.

29. A method for preparing a non-human mammal or progeny thereof, wherein the method comprises knocking out a nucleotide sequence, encoding a CH1 domain of an IgM heavy chain constant region, on a genome of the non-human mammal.

30. The preparation method according to claim 29, wherein the non-human mammal is a rodent; optionally, the rodent is a rat or a mouse; further optionally, the rodent is a mouse; and still further, the mouse is a C57BL/6 mouse or a BALB/c mouse.

31. The preparation method according to claim 29 or 30, wherein the non-human mammal or progeny thereof is used to construct the non-human mammal or progeny thereof according to claim 21.

32. The use of a non-human mammal or progeny thereof constructed by using the preparation method derived from any one of claims 1-3, a non-human mammal or progeny thereof constructed by using the preparation method derived from any one of claims 4-13, a C57BL/6 mouse or progeny thereof constructed by using the preparation method derived from any one of claims 14-20, or a C57BL/6 mouse or progeny thereof constructed by using the preparation method derived from any one of claims 21-28 in the screening of a target heavy chain antibody.

33. The use according to claim 32, wherein a phage display method is used when the target heavy chain antibody is screened; and
preferably, the screening of the target heavy chain antibody is screening of a C-reactive protein, coronavirus S protein or coronavirus N protein antigen-specific IgG2c heavy chain antibody.

34. A method for screening a target heavy chain antibody, wherein the method comprises performing screening by using a non-human mammal or progeny thereof constructed by using the preparation method derived from any one of claims 1-3, a non-human mammal or progeny thereof constructed by using the preparation method derived from any one of claims 4-13, a C57BL/6 mouse or progeny thereof constructed by using the preparation method derived from any one of claims 14-20, or a C57BL/6 mouse or progeny thereof constructed by using the preparation method derived from any one of claims 21-28 as an immune animal.

35. The method according to claim 34, wherein a phage display method is used when the target heavy chain antibody is screened; and
preferably, the screening of the target heavy chain antibody is screening of a C-reactive protein, coronavirus S protein or coronavirus N protein antigen-specific IgG2c heavy chain antibody.

36. A non-human mammal cell or cell line or primary cell culture, wherein the non-human mammal cell or cell line or primary cell culture is derived from a non-human mammal or progeny thereof constructed by using the preparation method of any one of claims 1-3, a non-human mammal or progeny thereof constructed by using the preparation method of any one of claims 4-13, a C57BL/6 mouse or progeny thereof constructed by using the preparation method of any one of claims 14-20, a C57BL/6 mouse or progeny thereof constructed by using the preparation method of any one of claims 21-28, or a non-human mammal cell or progeny thereof constructed by using the preparation method of any one of claims 29-31.

37. An in-vitro tissue or in-vitro organ or a culture thereof, wherein the in-vitro tissue or in-vitro organ or the culture thereof is derived from a non-human mammal or progeny thereof constructed by using the preparation method of any one of claims 1-3, a non-human mammal or progeny thereof constructed by using the preparation method of any one of claims 4-13, a C57BL/6 mouse or progeny thereof constructed by using the preparation method of any one of claims 14-20, a C57BL/6 mouse or progeny thereof constructed by using the preparation method of any one of claims 21-28, or a non-human mammal cell or progeny thereof constructed by using the preparation method of any one of claims 29-31.

38. An sgRNA composition, wherein the sgRNA composition comprises: sgRNA targeting a upstream of a first exon of a gene encoding an IgM heavy chain constant region in a mouse and sgRNA targeting a downstream of the first exon of the gene encoding an IgG2c heavy chain constant region in the mouse; or
sgRNA targeting the upstream and downstream of the first exon of the gene encoding the IgM heavy chain constant region in the mouse; or
sgRNA targeting the upstream of the first exon of the gene encoding the IgG3 heavy chain constant region in the mouse and sgRNA targeting the downstream of the first exon of the gene encoding IgG2c in the mouse.

39. The sgRNA composition according to claim 38, wherein a targeting sequence, targeted by sgRNA, on the upstream of the first exon of the gene encoding the IgM heavy chain constant region in the mouse comprises SEQ ID NO. 1 and SEQ ID NO. 2; and/or
the targeting sequence, targeted by sgRNA, on the downstream of the first exon of the gene encoding the IgG2c heavy chain constant region in the mouse comprises SEQ ID NO. 3 and SEQ ID NO. 4; and/or
the targeting sequence, targeted by sgRNA, on the downstream of the first exon of the gene encoding the IgM heavy chain constant region in the mouse comprises SEQ ID NO. 5 and SEQ ID NO. 6; and/or
the targeting sequence, targeted by sgRNA, on the upstream of the first exon of the gene encoding the IgG3 heavy chain constant region in the mouse comprises SEQ ID NO. 7 and SEQ ID NO. 8.

40. The sgRNA composition according to claim 38, wherein sgRNA targeting the upstream of the first exon of the gene encoding the IgM heavy chain constant region in the mouse is SEQ ID NO. 9 and SEQ ID NO. 10; and/or
sgRNA targeting the downstream of the first exon of the gene encoding the IgM heavy chain constant region in the mouse is SEQ ID NO. 13 and SEQ ID NO. 14; and/or
sgRNA targeting the upstream of the first exon of the gene encoding the IgG3 heavy chain constant region in the mouse is SEQ ID NO. 15 and SEQ ID NO. 16; and/or
the sgRNA targeting the downstream of the first exon of the gene encoding the IgG2c heavy chain constant region in the mouse is SEQ ID NO. 11 and SEQ ID NO. 12.

41. A knockout vector, comprising: DNA sequence encoding one or more segments of sgRNA, wherein a targeting sequence targeted by the sgRNA is selected from one of followings:
a targeting sequence on a upstream of a first exon of a gene encoding an IgM heavy chain constant region in a mouse; or
the targeting sequence on a downstream of the first exon of the gene encoding an IgG2c heavy chain constant region in the mouse; or
the targeting sequence on the downstream of the first exon of the gene encoding the IgM heavy chain constant region in the mouse; or
the targeting sequence on the upstream of the first exon of the gene encoding an IgG3 heavy chain constant region in the mouse.

42. The knockout vector according to claim 41, wherein a backbone of the knockout vector is an sgRNA expression vector.

43. A cell comprising the knockout vector of any one of claims 41-42.
